(19) Europäisches Patentamt
European Patent Office
Office européen des brevets

(11) **EP 1 938 754 A1**

(12) **EUROPEAN PATENT APPLICATION**
published in accordance with Art. 153(4) EPC

(43) Date of publication:
**02.07.2008 Bulletin 2008/27**

(51) Int Cl.:
**A61B 8/08** *(2006.01)*

(21) Application number: **06811533.6**

(22) Date of filing: **10.10.2006**

(86) International application number:
**PCT/JP2006/320221**

(87) International publication number:
**WO 2007/046272 (26.04.2007 Gazette 2007/17)**

(84) Designated Contracting States:
**DE FR GB IT NL**

(30) Priority: **19.10.2005 JP 2005304347**

(71) Applicant: **Hitachi Medical Corporation
Tokyo 101-0021 (JP)**

(72) Inventor: **MATSUMURA, Takeshi
Tokyo 101-0021 (JP)**

(74) Representative: **Strehl Schübel-Hopf & Partner
Maximilianstrasse 54
80538 München (DE)**

(54) **ULTRASONOGRAPH FOR CREATING ELASTIC IMAGE**

(57) Based on the ultrasonic tomographic data measured by exerting the pressure to the tissue of a body of the subject 1, the elastic data such as the distortion or the elastic modulus of the tissue at the plural measurement points in the tomographic site of the subject body are obtained. Based on the obtained elastic data, the elastic image in the tomographic site is generated to be displayed. The distribution of the elastic data such as the distortion and the elastic modulus in the interest region ROI set on the elastic image is displayed as the histogram. The new quantitative information which reflects the tissue property is supplied in addition to the information which relates to the elasticity of the tissue graded with the hue or brightness for assisting in the tissue identification.

## FIG. 8

**Description**

Technical Field

The present invention relates to an ultrasonograph for creating elastic image intended to assist in an appropriate diagnosis by supplying to users an elastic image generated based on the measured elasticity (rigidity or flexibility) of the body tissue of the subject resulting from the compression exerted thereto, and other quantitative information which relates to the elasticity of the tissue.

Background Art

In the technical field of the ultrasonic diagnostic system, as described in Patent Documents 1 and 2, there has been proposed that the compression is exerted to the subject with the ultrasonic transmission/reception surface of the ultrasonic probe manually or mechanically to obtain displacements of the respective sites in the body through the correlation calculation of the ultrasonic reception signals which closely exist in time-series. Then the elastic data with respect to the tissue elasticity are obtained based on the obtained displacements so as to create the image of the elastic data. The elastic modulus represented by Young's modulus of the body tissue derived from the displacements of the respective sites in the body, the distortion obtained through spatial differentiation, and the stress distribution and distortion inside the body resulting from the external compression may be used as the elastic data of the tissue. The aforementioned elastic data are measured to display the elastic image graded with the hue or brightness in accordance with the size of the data such that the elasticity of the body tissue may be easily recognized, and the diseased tissue such as the cancer tumor may be appropriately identified.

Since the elastic image is graded with the hue or brightness in accordance with the level of the elasticity, for example, the displacements of the respective measurement points, distortion or the elastic modulus, the rigidity or flexibility of the body tissue may be easily recognized.

In the case where the diseased site is identified by determining with respect to the difference in the elasticity of the target site based on the elastic image, it may be the case where the difference in the elasticity in the boundary region between different color gradients, or the region with similar color gradients cannot be clarified. In the aforementioned case, the operator with insufficient experience or skill may fail to have the correct determination.

Alternatively, the treatment method performed by irradiating the high-intensity focused ultrasound (hereinafter referred to as HIFU) to destroy the diseased tissue has also been proposed. As the destroyed diseased tissue will become rigid owing to the thermal denaturalization, the method allows the level of the rigidity to be correlated with the treatment effect. The determination whether the treatment has been performed uniformly may be made by measuring the elasticity in the site to be treated and observing the elastic image. However, as mentioned above, it is difficult to clearly identify the gradient difference with respect to the minimal portion in the region having similar color gradients in the elastic image. As a result, the determination whether the treatment has been performed cannot be clearly made.

In the case where the diseased site is identified based on the elastic image, or the treatment effect is determined, other quantitative information relative to the tissue elasticity in addition to the elastic information such as the color gradient may be helpful to assist the operator in the appropriate diagnosis.

Patent Document 1: JP 5-317313 A
Patent Document 2: JP 2000-60853 A

Disclosure of Invention

The present invention provides the quantitative auxiliary elastic information in addition to the elastic image of the tissue graded with hue or brightness for assisting in the appropriate diagnosis.

In the course of the consideration for solving the above-identified problems, it is confirmed firstly that the tissue deformation with the external force such as compression exerted thereto causes the tissue-forming elements to have inherent behavior in unison. It is assumed that if the inherent unison behavior may be expressed as the statistical data, they may be the auxiliary elastic information data for assisting in the appropriate diagnosis.

To solve the above-identified problems, the present invention provides the ultrasonic diagnostic system which includes elastic data calculation means for calculating elastic data of the tissue at plural measurements points in a tomographic site of a subject based on an ultrasonic tomographic data measured by compressing the tissue of the subject, elastic image generation means for generating an elastic image in the tomograhpic site based on the elastic data, histogram evaluation means for generating a histogram showing an elasticity distribution based on the elastic data, and image display means for displaying at least one of the elastic image and the histogram image. In this case, an interest region is set on the elastic image such that the elastic distribution in the interest region is formed into the histogram.

The distribution of the elastic data such as the displacement, distortion or elastic modulus in the interest region including the target site may be displayed as the histogram. Accordingly, the quantitative auxiliary information based on the unison behavior which reflects the tissue property may be supplied. The histogram is a graph showing the number of the measurement points with identical or similar elastic data by defining the elastic data such as the displacements, distortion, or elastic modulus of the tissue at all the measurement points in the interest region as the population. The operator observes the histogram to identify the elastic distribution of the tissue in the interest region including the target site quantitatively and relatively. The tissue identification at the diseased site may be effectively assisted. The determination with respect to the treatment effect may also be appropriately made.

Particularly, as the histogram represents the feature of the unison behavior of the tissue, the evaluation may be appropriately made with respect to the uniformity in the elasticity of the tissue at the target site such as the diseased site. This makes it possible to identify the various types of the tissue properties, for example, the cancer tumor as considerably rigid solid tissue, the fiber adenoma as the flexible solid tissue, or the cyst as the mobile cystic tissue so as to provide the clinically useful diagnostic information.

In the aforementioned case, the interest region set on the'elastic image may be automatically changed to follow the tissue in the interest region which displaces in accordance with the pressure exerted to the subject. This makes it possible to correctly recognize how the distribution of the elastic information of the tissue is changed at any time.

The tomographic image in the tomographic site generated based on the ultrasonic tomographic data, the elastic image, and the histogram may be displayed on the same screen. The distribution image of the tissue shown on the tomographic image, the elastic information of the respective regions based on the elastic image, and the auxiliary elastic information based on the histogram may be observed simultaneously, thus further improving the accuracy in the tissue identification.

The histogram image may contain not only the histogram but also the property derived from the histogram as the auxiliary elastic information. For example, the histogram image includes at least one of the average, the standard deviation, and the median of the elastic data. At least one of the functions by approximating the histogram distribution, the elastic data of the peak in the histogram, the standard deviation of the peak, and the half width of the peak may be displayed together with the histogram. A cursor is displayed on the image of the histogram such that the number of the measurement points in a single section of the histogram designated by the cursor, and the elastic data in the section may be displayed as the numeric values. Plural cursors may be displayed on the histogram image such that at least one of the number of the measurement points in the plural sections of the histogram between two cursors, the average value of the elastic data in the plural sections, the standard deviation of the elastic data in the plural sections, and the half value of the peak may be displayed in the numeric value.

The tomographic image in the tomographic site generated based on the ultrasonic tomographic data, the elastic image, and the histogram is displayed on the same screen, and plural cursors are displayed on the histogram image. At least one of the number of the measurement points in the plural sections of the elastic data between the two cursors, the average value of the elastic data in the plural sections, and the standard deviation of the elastic data in the plural sections is displayed in the numeric value as the auxiliary elastic information. Positions of the plural measurement points in the section between the two cursors may be displayed on the elastic image so as to be identifiable.

The plural cursors are displayed on the histogram image such that the ratio of the elastic data in the two elastic data sections designated by the cursors is displayed in the numeric value as the auxiliary elastic information. This makes it possible to evaluate the elasticity of the tissue at two target sites with respect to the relative ratio.

The cursor is displayed on the histogram image, and the half width of the elastic data of the peak in the histogram distribution designated by the cursor is obtained. Based on the obtained half width value, the tissue property corresponding to the peak is evaluated to display the evaluation result as the auxiliary elastic information together with the histogram.

The interest region set on the elastic image may be automatically changed by following the tissue which displaces in accordance with the pressure, and the histogram in the changed interest region may be displaced correspondingly.

The ultrasonic diagnostic system according to the present invention is capable of obtaining the displacement vector of the tissue at the plural measurement points in the tomographic site of the subject based on the ultrasonic tomogrpahic data measured under the pressure to the tissue of the subject, generating the elastic image for displaying the displacement vector corresponding to the measurement points at the tomographic site so as to be displayed, and displaying the distribution of the displacement vector component in the specific direction in the interest region set on the elastic image as the histogram.

In the present invention, besides the elastic image of the tissue graded with hue or brightness, the histogram indicating the elastic distribution of the tissue in the interest region is displayed as the auxiliary elastic information. This makes it possible to quantitatively and relatively identify the elastic distribution of the tissue in the interest region which contains the target site for effectively assisting in identification of the tissue at the diseased site.

Brief Description of the Drawings

Fig. 1 is a block diagram of an ultrasonic diagnostic system according to an embodiment of the present invention.
Fig. 2 is a flowchart according to a control routine in the ultrasonic diagnostic system of an embodiment of the present invention.
Fig. 3(A) is an outline view of an exemplary ultrasonic probe.
Fig. 3(B) is an outline view of an exemplary ultrasonic probe provided with a compression plate.
Fig. 3(C) is an outline view of an exemplary ultrasonic probe having pressure sensors attached to the compression plate.
Fig. 3(D) is an outline view of an exemplary ultrasonic probe having a reference deforming body attached to the compression plate.
Fig. 4 is a view showing the structure of a histogram evaluation unit according to an embodiment of the invention.
Fig. 5 is an explanatory view showing the concept of the elastic frame data.
Fig. 6 is an explanatory view showing the elastic image according to an embodiment of the present invention.
Fig. 7 is an explanatory view with respect to ROI set on the elastic frame data.
Fig. 8 is a view showing an exemplary display of a histogram according to a first embodiment of the present invention.
Fig. 9 is a view showing an exemplary display of the image according to the present invention.
Fig. 10 is a view showing an exemplary display of a histogram according to a fourth embodiment of the present invention.
Fig. 11 is a view showing an exemplary display of a histogram according to a fifth embodiment of the present invention.
Fig. 12 is a view showing an exemplary display of a histogram according to a sixth embodiment of the present invention.
Fig. 13 is a view showing an exemplary display of a histogram according to a seventh embodiment of the present invention.
Fig. 14 is a view showing an exemplary display of a histogram according to an eighth embodiment of the present invention.
Fig. 15 is a view showing an exemplary display of a histogram according to a tenth embodiment of the present invention.
Fig. 16 is a view showing the display example of the histogram for comparison with the one in the tenth embodiment.
Fig. 17(A) is a view showing an exemplary display of a histogram according to a fourteenth embodiment of the present invention.
Fig. 17(B) is a view showing another exemplary display of the histogram according to the fourteenth embodiment of the present invention.
Fig. 17(C) is a view showing still another exemplary display of the histogram according to the fourteenth embodiment of the present invention.
Fig. 18 is a view showing an exemplary display of a histogram according to a fifteenth embodiment of the present invention.
Fig. 19 is a view showing an exemplary display of a histogram according to a sixteenth embodiment of the present invention.
Fig. 20 is a view showing another exemplary display of a histogram according to the sixteenth embodiment of the present invention.
Fig. 21 is a view showing an exemplary display of a histogram according to a seventeenth embodiment of the present invention.
Fig. 22 is a view showing an exemplary display of a histogram according to an eighteenth embodiment of the present invention.
Fig. 23 shows an exemplary display of a histogram according to a nineteenth embodiment of the present invention.
Fig. 24 shows an exemplary display of a histogram according to a twentieth embodiment of the present invention.
Fig. 25 shows an exemplary display of a histogram according to a twenty-first embodiment of the present invention.
Fig. 26 shows an exemplary display of a histogram according to a twenty-second embodiment of the present invention.

Best Mode for Carrying out the Invention

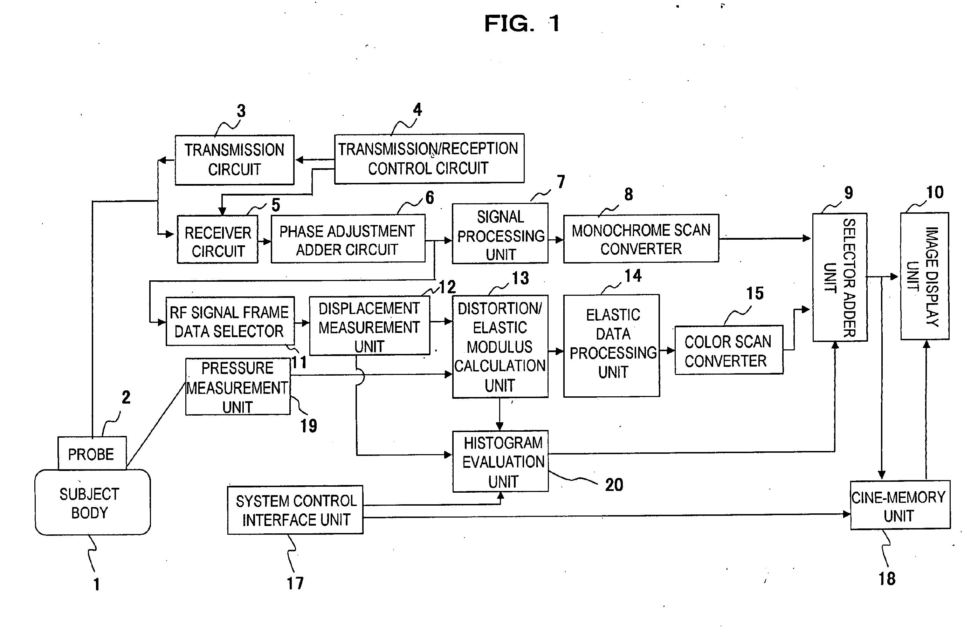

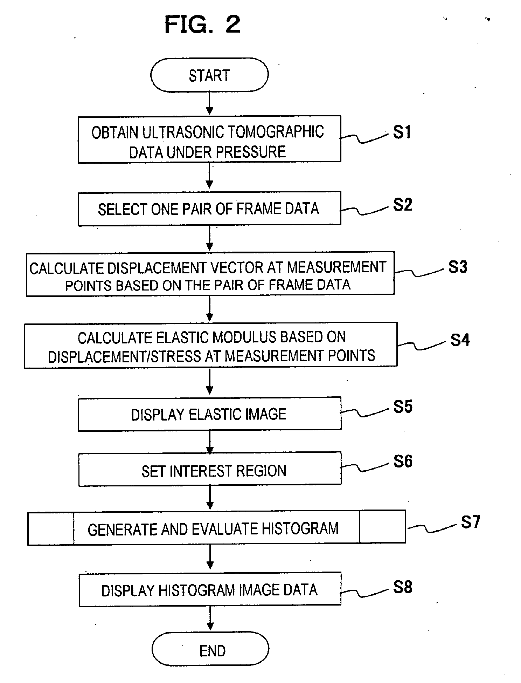

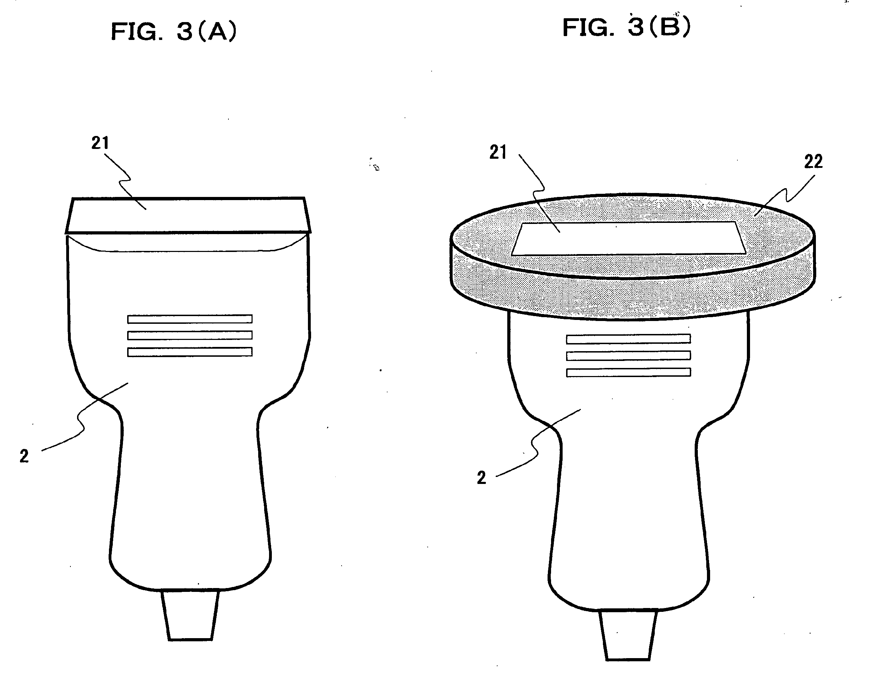

The present invention will be described based on the embodiments. Fig. 1 is a block diagram of an ultrasonic diagnostic system according to an embodiment of the invention. Fig. 2 is a flowchart of the control routine executed in the ultrasonic diagnostic system according to an embodiment as the feature of the invention.
Referring to Fig. 1, an ultrasonic probe 2 to be pressed against a subject 1 includes an ultrasonic transmission/reception surface 21 having plural transducers aligned for transmitting/receiving the ultrasonic wave to/from the subject 1 as shown in Fig. 3(A). The probe 2 is driven by the ultrasonic pulse fed from a transmission circuit 3 for mechanically or electronically scanning beam. A transmission/reception control circuit 4 controls the timing for transmitting the ultrasonic pulse to drive the plural transducers on the probe 2 for forming the ultrasonic beam to the focal point set in the subject 1. The transmission/reception control circuit 4 is structured to electronically scan the ultrasonic beam in the direction of alignment of the transducers of the probe 2.

Meanwhile, the probe 2 receives a reflection echo signal generated in the subject 1 so as to be output to a receiver circuit 5. The receiver circuit 5 executes the reception process by taking the reflection echo signal for amplification in accordance with the timing signal input from the transmission/reception control circuit 4. The reflection echo signal subjected to the reception process by the receiver circuit 5 controls the phase of the reflection echo signal received by the plural transducers in a phase adjustment adder circuit 6 to form the ultrasonic wave reception beam with respect to at least one convergence point. The reflection echo signal subjected to the phase adjustment and addition in the phase adjustment adder circuit 6 (hereinafter referred to as the ultrasonic tomographic data) is input to a signal processing unit 7 where signal processing such as the gain correction, log compression, phase detection, outline highlighting, filter processing and the like is performed. The high frequency (RF) signal of the ultrasonic tomographic data generated in the phase adjustment adder circuit 6 may be the combined and demodulated I, Q signals. The ultrasonic beam is scanned in one direction inside the subject 1 by the probe 2 so as to obtain the ultrasonic tomographic data corresponding to a single sheet of the tomographic image.

The ultrasonic tomographic data processed in the signal processing unit 7 is led to a monochrome scan converter 8 to be converted into the digital signal and further converted into the two-dimensional tomographic image data corresponding to the scan surface of the ultrasonic beam. The monochrome scan converter 8 serves as means to control the tomographic scan means and the system for reading at TV sinc. such that the RF signal frame data inside the subject 1 including the mobile tissue are derived at the ultrasonic frequency, and the frame data are converted into the image to be displayed. The monochrome scan converter 8 includes an A/D converter for converting the reflection echo signal from the signal processing unit 7 into the digital signal, a frame memory which stores the plural sheets of the tomographic data digitized in the A/D converter in time-series, and the controller for controlling the operation of the aforementioned components. The signal processing unit 7 and the monochrome scan converter 8 form the means for restructuring the image of the tomogrpahic image. The tomogrpahic image data output from the monochrome scan converter 8 are supplied to an image display unit 10 via a selector adder unit 9 such that the tomographic image is displayed.

The image display unit 10 for displaying the time-series tomographic data obtained by the monochrome scan converter 8 includes a D/A converter for converting the image data input via a selector adder unit 9 into the analog signal, and a color TV monitor which receives the input of the analog video signal from the D/A converter so as to be displayed as the image.

Meanwhile, the ultrasonic tomographic data output from the phase adjustment adder circuit 6 are led to the RF signal frame data selector 11 which selects the RF signal groups corresponding to the scan surface (tomographic surface) of the ultrasonic beam for the plural frames as the frame data so as to be stored in the memory. A displacement measurement unit 12 sequentially takes plural pairs of the frame data stored in the RF signal frame data selector 11 at different time points, and obtains the displacement vector at the plural measurement points on the tomogrpahic surface based on the taken pair of frame data so as to be output to a distortion/elastic modulus calculation unit 13 as the displacement frame data.

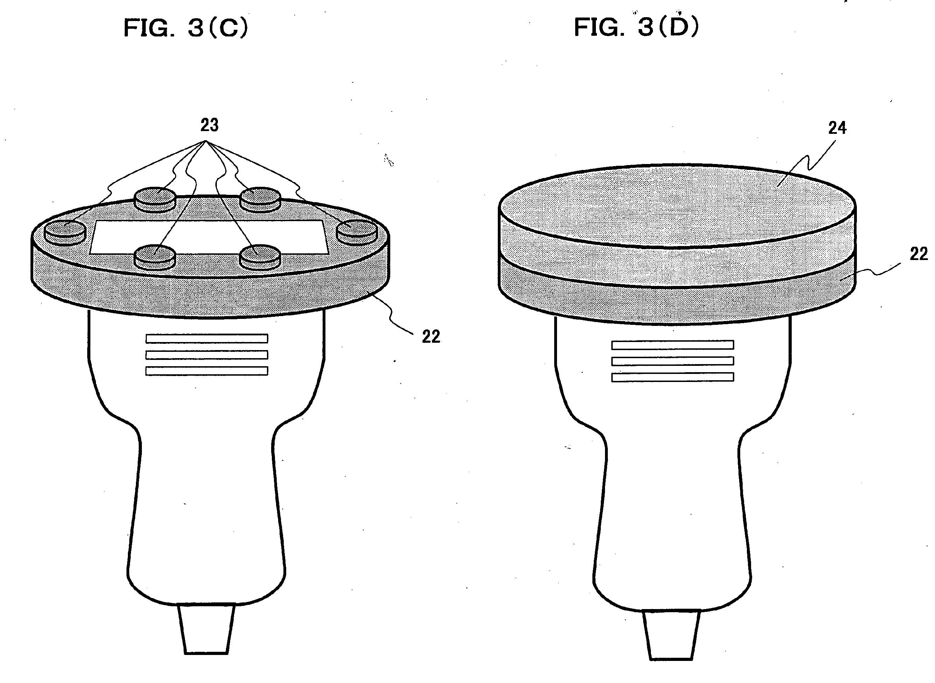

The distortion/elastic modulus calculation unit 13 obtains the distortion at the plural measurement points on the tomographic surface based on the input displacement frame data so as to be output to an elastic data processing unit 14 as the elastic frame data. The distortion/elastic modulus calculation unit 13 takes measurement data of the pressure exerted to the subject from a pressure measurement unit 19 to obtain the stress distribution at the respective portions of the subject. The elastic modulus is derived from the stress distribution and the distortion frame data which have been already obtained. The elastic modulus is output to the elastic data processing unit 14 as the elastic frame data. As shown in Fig.3(C), the pressure measurement unit 19 takes output signals from the plural pressure sensors 23 attached to the compression plate 22 to measure the pressure exerted to the surface of the body of the subject 1. The measured pressure data are transmitted to the distortion/elastic modulus calculation unit 13. As shown in Fig. 3(D), the pressure exerted to the body surface may be measured based on the displacement frame data of a reference deforming body 24 using the probe 2 equipped with the reference deforming body 24 attached on the upper surface of the compression plate 22. The pressure measurement process is disclosed in Japanese Unexamined Patent Application Publication No. 2005-13283 or 2005-66041.

The elastic data processing unit 14 subjects the elastic frame data like the distortion or the elastic modulus input from the distortion/elastic modulus calculation unit 13 to various image processing such as the smoothing process in the coordinate plane, the contrast optimizing process, and the smoothing process between the frames in the time-axis direction. The processed signal will be transmitted to a color scan converter 15.

The color scan converter 15 generates a color elastic image by converting the elastic frame data output from the elastic data processing unit 14 so as to be displayed on the image display unit 10 through the selector adder unit 9. That is, the color scan converter 15 applies the graded hue code such as red, green, and blue (for example, 256 gradients) to the elastic image based on the predetermined ranges of the upper and the lower limits of the elasticity (distortion or elastic modulus). For example, the rigid region where the elastic modulus of the elastic frame data measures the large value is converted into the blue code, and the flexible region where the elastic modulus measures the small value is converted into the red code. The monochrome scan converter may be used instead of the color scan converter 15. In the aforementioned case, the distribution of the elastic modulus may be expressed by the brightness, that is, the brightness of the rigid region

where the elastic modulus measures the large value may be made high, and the brightness of the flexible region where the elastic modulus measures the small value may be made low.

The selector adder unit 9 includes a function for receiving the monochrome tomographic data output from the monochrome scan converter 8, and color elastic image data output from the color scan converter 15 to select one of those data to be displayed, a function for making one of those image data half-transparent to be subjected to the additive synthesis and superimposing the resultant image on the image display unit 10, and a function for displaying both the image data side by side. A cine-memory unit 18 stores the image data output from the selector adder unit 9 in the memory and calls the previous image data in response to the command from the system control interface unit 17 so as to be displayed on the image display unit 10. The selected image data may be transferred to such recording medium as MO.

A histogram evaluation unit 20 as a feature of the embodiment takes the displacement frame data or the elastic frame data such as the distortion frame data output from the displacement measurement unit 12 and the distortion/ elastic modulus calculation unit 13 to generate the histogram, as described below, and evaluates the statistic characteristics based on the histogram. The histogram image data are generated to be displayed on the image display unit 10 via the selector adder unit 9.

The detail structure of the aforementioned embodiment will be described accompanied with the operation referring to the flowchart shown in Fig. 2.

(Step S1)

The ultrasonic tomographic data are sequentially obtained while exerting the pressure to the tissue of the subject 1 (S1). At this time, the ultrasonic transmission/reception surface 21 of the probe 2 as shown in Fig. 3(A) is brought into contact with the body surface of the subject 1 for transmitting/receiving the ultrasonic wave while being moved up and down manually to compress the subject 1. Preferably, as described in Fig. 3(B), in order to apply effectively the stress distribution to the diagnostic point of the subject 1, the compression plate 22 is attached to the ultrasonic transmission/reception surface 21 of the probe 2 having both surfaces on the same plane to press the body of the subject 1 with the compression surface formed of the ultrasonic transmission/reception surface 21 and the compression plate 22.

(Step S2)

The RF signal frame data selector 11 sequentially stores the ultrasonic tomographic data (RF signal frame data) sequentially output from the phase adjustment adder circuit 6 in time-series at the frame rate of the ultrasonic diagnostic system in the frame memory. The latest RF signal frame data are stored as N. In response to the control command, one of the RF signal frame data X is selected among the previous RF signal frame data N-1, N-2, N-3, ..., N-M. Then the latest RF signal frame data N and the selected RF signal frame data X are combined as a pair of RF signal frame data so as to be output to the displacement measurement unit 12.

(Step S3)

The displacement measurement unit 12 subjects the selected pair of the RF signal frame data N and X to the one- or two-dimensional correlation processing to measure each displacement or displacement vector (direction and amount of the displacement) of the respective measurement points on the tomographic image to generate the displacement frame data. As the process for detecting the displacement vector, the block matching process and gradient process may be employed as disclosed in Japanese Unexamined Patent Application Publication No. 5-317313. In the block matching process, the image is divided into plural blocks formed of n x n pixels, and the block which is the most approximate to the target block in the present frame is searched among the blocks in the previous frame such that the displacement vector is obtained.

(Step S4)

The distortion/elastic modulus calculation unit 13 calculates each distortion and elastic modulus of the respective measurement points on the tomographic image based on the displacement frame data and the pressure data derived from the displacement measurement unit 12 and the pressure measurement unit 19, respectively. The elastic frame data as the distortion or the elastic modulus corresponding to the tomographic image are generated to be output to the elastic data processing unit 14. The distortion is calculated by performing spatial differentiating of the displacement with no need of the pressure data. One of the elastic modulus, for example, Young's modulus is calculated by obtaining each stress (pressure) at the respective measurement points based on the pressure data as shown in the following formula, and further dividing the stress by each distortion amount at the respective measurement points. The indexes of i and j denote the coordinate of the measurement point of the frame data.

$$Ymi,j = pressure (stress)ij/(distortion amount i,j)$$

where i,j = 1, 2, 3, ...

(Step S5)

The elastic data processing unit 14 subjects the elastic frame data input from the distortion/elastic modulus calculation unit 13 to the image processing, for example, the smoothing processing in the coordinate plane, the contrast optimizing process, the smoothing process between the frames in the time axis direction and the like such that the processed data are transmitted to the color scan converter 15. The image processing performed by the elastic data processing unit 14 is disclosed in Japanese Unexamined Patent Application Publication No. 2004-261198, for example.
The color scan converter 15 inputs the elastic frame data output from the elastic data processing unit 14, and the upper and the lower limit values for defining the gradient selection range of the elastic frame data to generate the elastic image data formed by converting the elastic frame data into the hue information. The upper and the lower limit values are defined by the command from the system control unit (not shown) or the gradient selection range in the elastic frame data from the elastic data processing unit 14. For example, the color scan converter 15 converts the region where the large elastic modulus is detected into the blue code, and converts the region where the small elastic modulus is detected into the red code on the input elastic frame data.
The monochrome scan converter may also be used instead of the color scan converter 15. It may be structured to increase the brightness of the region where the large distortion is detected, and decrease the brightness of the region where the small distortion is detected.
The elastic image data generated by the color scan converter 15 are output to the selector adder unit 9. The selector adder unit 9 sums the monochrome tomographic image data output from the monochrome scan converter 8 and the color elastic image data output from the color scan converter 15, or selects one of those data so as to be output to the image display unit 10 for display. For example, either the monochrome tomographic image data or the color elastic image data may be displayed, or both of those data are summed and synthesized to be displayed. As disclosed in Japanese Unexamined Patent Application Publication No. 2000-60853, the monochrome tomographic image and the color or monochrome elastic image may be displayed on the respective two screens simultaneously. As disclosed in Japanese Unexamined Patent Application Publication No. 2004-135929, the half-transparent color elastic image may be superimposed onto the monochrome tomographic image so as to be displayed.
The display image data output from the selector adder unit 9 are transmitted to the image display unit 10, and input to the cine-memory unit 18 simultaneously. The cine-memory unit 18 stores the input display image data into the memory. The cine-memory unit 18 calls the previous display image data to be displayed on the image display unit 10 or transfers the selected display image data to the recording medium such as the MO in accordance with the control signal from the system control interface unit 17.

(Steps S6 and S7)

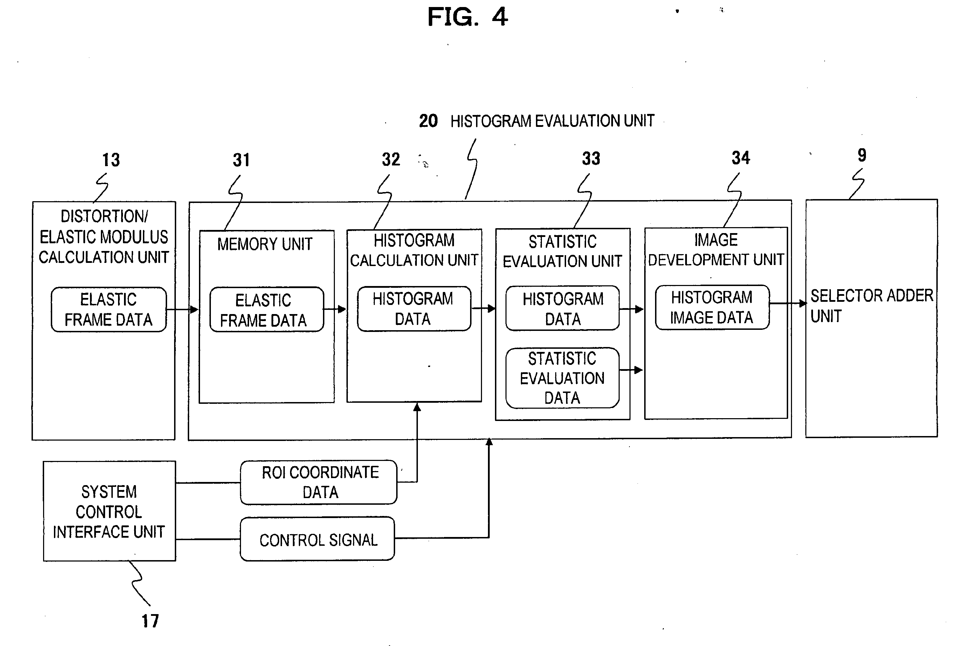

In steps S6 and S7, the histogram evaluation processing according to the present invention is executed. The histogram evaluation unit 20 obtains the distortion and elastic modulus at the respective measurement points in the interest region at the current time using the elastic frame data output from the distortion/elastic modulus calculation unit 13 to obtain the histogram in the interest region. The image information which reflects the obtained histogram is generated to be displayed on the image display unit 10 via the selector adder unit 9 for the purpose of informing the operator of the diagnostic information. The image information which reflects the histogram is allowed to contain the statistic feature of the elasticity at the respective sites in the interest region including the target site.
The flow of the process executed in the histogram evaluation unit 20 will be described referring to Fig. 4.. The histogram evaluation unit 20 includes a memory unit 31, a histogram calculation unit 32, a statistic evaluation unit 33, and an image development unit 34. The elastic frame data output from the distortion/elastic modulus calculation unit 13 are stored once in the memory unit 31, and then output to the histogram calculation unit 32.

The histogram calculation unit 32 receives the elastic frame data output from the memory unit 31, and the coordinate data of the interest region (ROI) output from the system control interface unit 17. The ROI is set to be input (S6) from the system control interface unit 17 on the color elastic image displayed on the image display unit 10 in step S5.

The histogram calculation unit 32 calculates the histogram by making the elastic frame data which distribute in the ROI as the population, and generates the histogram data including the numeric data so as to be output to the statistic evaluation unit 33. In other words, the distortion value or the elastic modulus value in the ROI is divided into plural sections, and the number of measurement points of the distortion value or the elastic modulus value in the respective sections is calculated. The histogram data are generated by allocating the plural sections of the distortion or the elastic modulus on the horizontal axis, and the number of the measurement points on the vertical axis.

The statistic evaluation unit 33 analyzes the histogram data output from the histogram calculation unit 32 to extract the statistic property of the histogram data. The statistic evaluation data as the statistic property are generated to be output to the image development unit 34 together with the histogram data. The image development unit 34 develops the statistic evaluation data output from the statistic evaluation unit 33 and the image which reflects the histogram data into the histogram image data so as to be output to the selector adder unit 9.

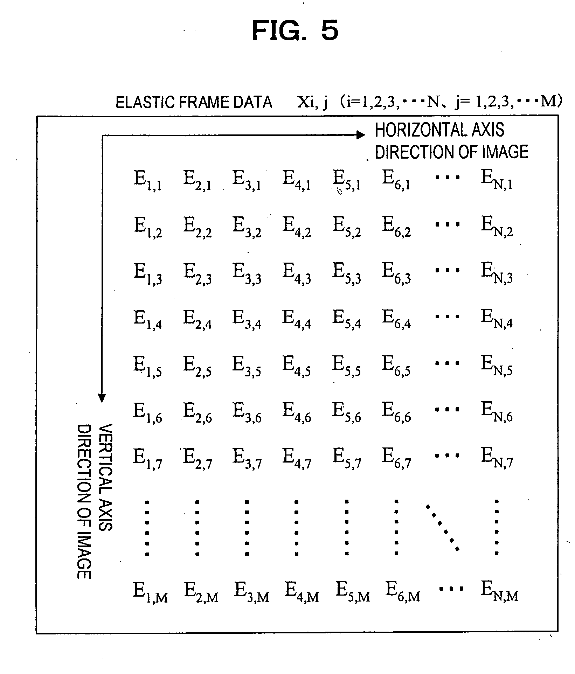

The process executed in the histogram evaluation unit 20 will be described in detail referring to Figs. 5 to 8. The numeric values of the elastic modulus in the elastic frame data at the time point t when the calculation is executed in the distortion/elastic modulus calculation unit 13 are defined to $E_{i,j}(t)$ where each of i (=1, 2, 3,...N) and j(=1, 2, 3,...M) denotes a natural number as shown in the conceptual view of Fig. 5. Referring to Fig. 5, the index i denotes the coordinate of the elastic image in the horizontal axis direction, and the index j denotes the coordinate of the elastic image in the vertical axis direction, respectively.

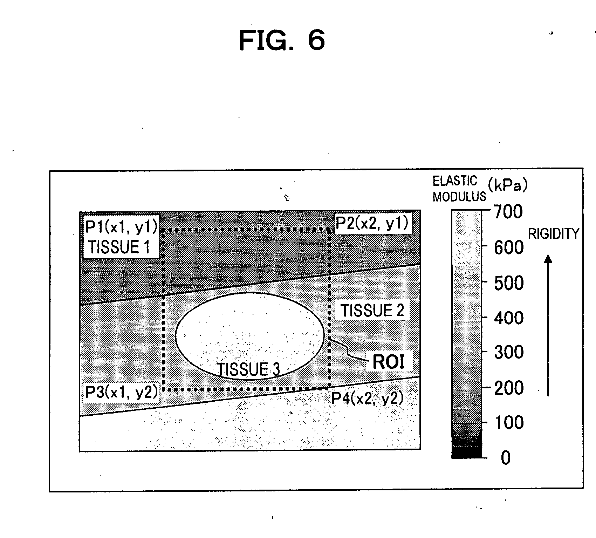

The memory unit 31 stores the elastic frame data $E_{i,j}(t)$ to be output to the histogram calculation unit 32. Meanwhile, the operator sets the interest region ROI via the input means of the system control interface unit 17 such device as a track ball on the elastic image displayed on the image display unit 10 as shown in Fig. 6. The system control interface unit 17 reads the coordinates of the set ROI, for example, four angular coordinates P1(x1, y1), P2(x2, y1), P3 (x1, y2), and P4 (x2, y2) in case of the rectangular ROI so as to be output to the histogram calculation unit 32 as the ROI coordinate data as shown in Fig. 6.

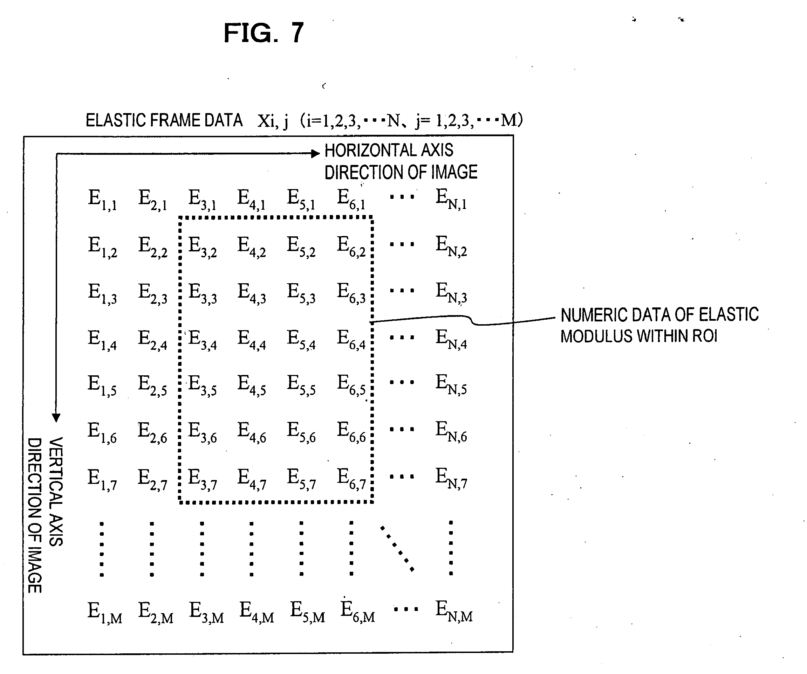

The histogram calculation unit 32 calculates the histogram data using the elastic frame data and the ROI coordinate data. First of all, the numeric data of the elastic modulus at the measurement points inside the ROI are extracted from the elastic frame data in reference to the ROI coordinate data as shown in Fig. 7.

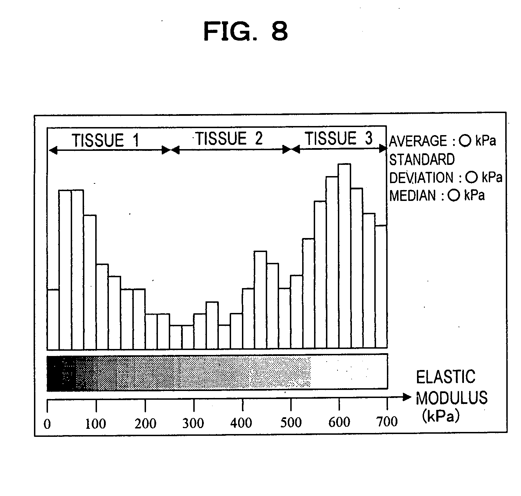

The histogram data are calculated by defining the numeric data of the elastic modulus at the measurement points in the ROI as population. The process for calculating the histogram data is the same as that for developing the general histogram. The process will be described in more detail referring to Fig. 8. The display range of the histogram is defined from 0 (kPa) to 700 (kPa) in terms of the elastic modulus, and the pin width as the section of the histogram is defined to 25 (kPa). Then 28 pins are provided, that is, each width of the respective pins is defined as follows.

    Pin 1 : 0-24.99 ... (kPa)
    Pin 2 : 25-49.99 ... (kPa)
    Pin 3 : 50-74.99 ... (kPa)
    ...
    Pin 27 : 650-674.99 ... (kPa)
    Pin 28 : 675-699.99 ... (kPa)

The number of the measurement points with the elastic modulus of the respective pins in the range is counted from the population corresponding to the thus set pins as follows.

    Pin 1 : 17 (counts)
    Pin 2 : 87 (counts)
    Pin 3 : 142 (counts)
    ...
    Pin 27 : 74 (counts)
    Pin 28 : 63 (counts)

The aforementioned processing is executed to generate the histogram data which reflect the relationship between the pin number (pin range) and the number of the measurement points.

The statistic evaluation unit 33 calculates the statistic feature of the generated histogram data to generate the statistic evaluation data. One of the number of the measurement points as the sample of the elastic modulus data as the population, the average value, the median, the distributed value, standard deviation of the elastic modulus data, half-width of the peak, distortion, and kurtosis may be selected to be set arbitrarily as the statistic features. It is to be understood that the property which represents the statistic feature is not limited to those described above.

(Step S8)

The image development unit 34 develops the histogram image data based on the histogram data calculated by the histogram calculation unit 32 and the statistic evaluation data obtained by the statistic evaluation unit 33. The developed histogram image data are displayed on the image display unit 10 via the selector adder unit 9 so as to be supplied to the operator as the auxiliary elastic information for the tissue identification.

In the embodiment, the measurement is made by contacting the probe 2 with the surface of the body of the subject 1. However, the invention is not limited to the one as described above. The invention is applicable to the arbitrary ultrasonic probe, for example, the rectum probe, transesophageal probe, the intraoperative probe, and the endovascular probe.

The embodiment of the histogram image data generated by the histogram evaluation unit 20 will be described. The case where the ROI is set as shown in Fig. 6 will be described in following embodiments.

First Embodiment

A first embodiment relates to generation of the histogram image as shown in Fig. 8. Referring to Fig. 6, ROI contains the tissues 1, 2, and 3. The information with respect to the rigidity inherent to the respective tissues 1 to 3 is displayed in real time on the image display unit 10 as the elastic image together with the ranges of the tissues 1 to 3. This makes it possible to easily recognize the relationship of (rigidity of tissue 1) < (rigidity of tissue 2) < (rigidity of tissue 3).

The histogram image as shown in Fig. 8 is generated based on the elastic modulus data in the ROI. That is, the vertical axis of the histogram image in the drawing correlates with the number of the measurement points belonging to the elastic modulus of the respective pins. As the number of the measurement points becomes large, the length of the pin increases. The population in the elastic frame data designated by the ROI contains elastic modulus data inherent to the respective tissues 1 to 3. The resultant histogram has the large number of the measurement points in the range of the corresponding elastic modulus.

The statistic evaluation unit 33 calculates the number of the measurement points belonging to the population, the average value, the median, and the variance of the elastic modulus in the population, which are displayed in real time on the image display unit 10 together with the histogram as shown in Fig. 8.

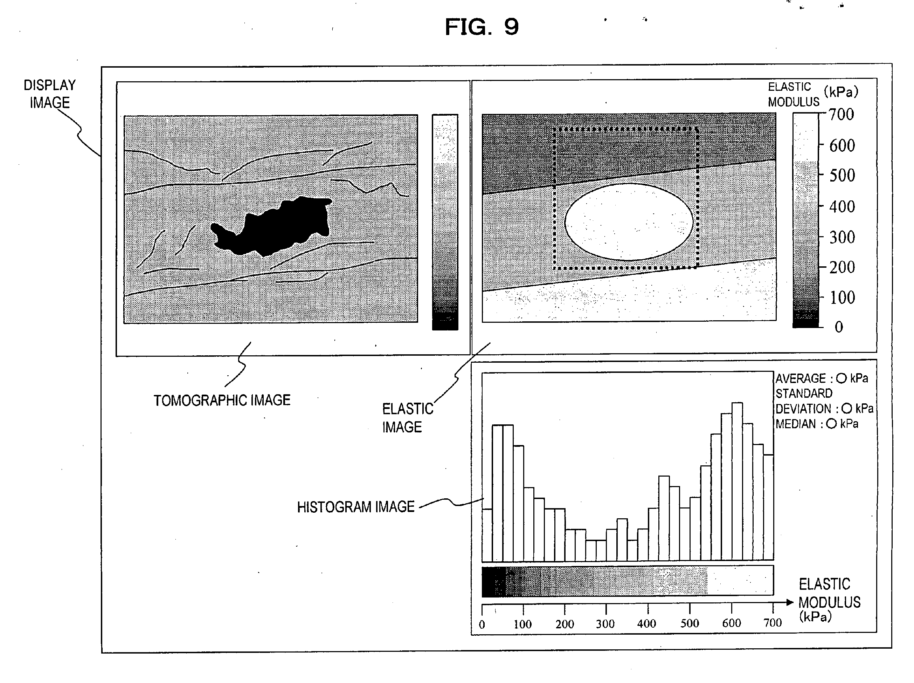

The selector adder unit 9 displays the image on the image display unit 10 in various display modes as shown in Fig. 9. Referring to the exemplary drawing, the tomographic image output from the monochrome scan converter 8, the elastic image output from the color scan converter 15, and the histogram image output from the histogram evaluation unit 20 are combined to develop the single display image data to be displayed in real time on the image display unit 10.

The embodiment allows the operator to evaluate the binding of the target region to the peripheral tissue in terms of rigidity in the objective and quantitative manner, which makes sure to assist in the tissue identification.

In the case of the elastic image showing the level of the brightness (hue) and the extent thereof, the diagnosis tends to be made by the operator subjectively. As a result, the diagnostic results vary depending on the operators. The embodiment allows the operator to diagnose in the objective and universal manner rather than the subjective manner, thus providing the clinically useful ultrasonic diagnostic system.

As the histogram reflects the feature of the unison behavior of the tissue, the uniformity in the rigidity of the tissue of the target site such as the diseased portion may be evaluated. This makes it possible to identify the tissue properties, for example, the cancer tumor as the considerably rigid solid tissue, the fiber adenoma'as the flexible solid tissue, or cyst as the mobile cystic tissue, thus providing the clinically useful diagnostic information.

Second Embodiment

In the first embodiment, the elastic modulus is employed. However, the present invention is not limited to the elastic modulus. The distortion data, displacement data, and the displacement vector may be employed as the elastic data. That is, the same histogram and the same statistic property data are obtained based on distortion as the elastic frame data calculated in the distortion/elastic modulus calculation unit 13 such that the image is displayed. Alternatively, the histogram and the statistic property data are obtained based on the displacement frame data calculated by the displacement calculation unit 12 such that the image is displayed. If the displacement frame data are formed of the displacement vectors, the histogram and the statistic property data are obtained based on the vector data such that the image is displayed.

The histogram and the statistic property data are obtained based on the elastic data correlating with the rigidity of the tissue so as to be displayed, thus providing substantially the same effect as that of the first embodiment.

Third Embodiment

In the first embodiment, the histogram and the statistic property data are obtained based on the elastic frame data formed of the numeric elastic modulus data calculated by the distortion/elastic modulus calculation unit 13. However, the present invention is not limited to one as described above, the histogram and the statistic property data may be obtained by inputting the elastic frame data subsequent to the image processing in the elastic data processing unit 14 into the histogram evaluation unit 20 such that substantially the same effect as that of the first embodiment may be obtained.

The histogram and the statistic property data may be obtained based on the elastic image data converted by the color scan converter 15 so as to provide the same effect as that of the first embodiment.

Fourth Embodiment

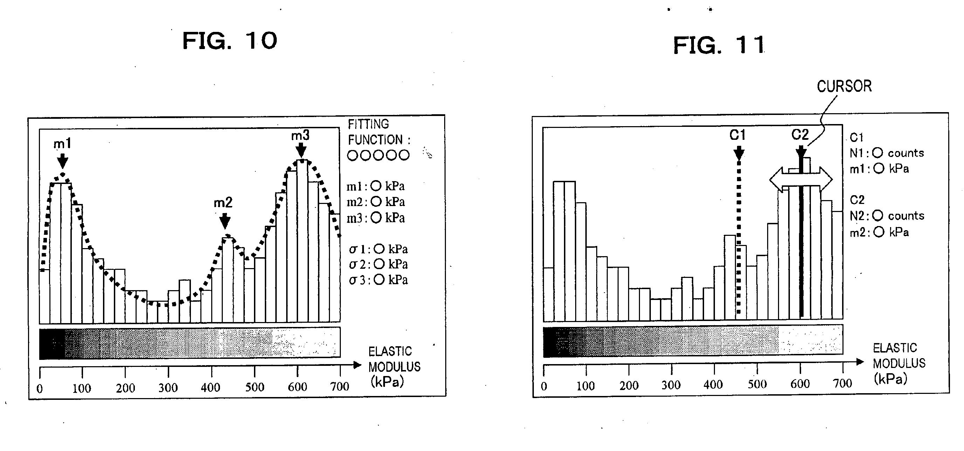

Fig. 10 shows an exemplary display of the histogram image according to a fourth embodiment. In the first embodiment, the histogram is generated based on the elastic modulus as the elastic data correlated with the elasticity so as to be displayed. The present invention is not limited to the one as described above. The obtained histogram is approximated (fitting) with an appropriate function such as double Gaussian or plural Gaussian functions by the statistic evaluation unit 33 so as to be displayed as shown in Fig. 10. In this case, the average and the standard deviation of the respective Gaussian function are obtained, and output as the statistic evaluation data. Referring to the drawing, the fitting function, the elastic modulus m1 to m3 at the peak of the function, or the average M of the elastic modulus and the width information such as the standard deviations σ1 to σ3 may be obtained to be displayed as the property amount data of the histogram.

Compared with the first embodiment, the embodiment further allows the operator to evaluate the binding of the target region with the peripheral tissue in terms of the rigidity in the objective and quantitative manner, and makes sure to assist in the tissue identification.

Fifth Embodiment

Fig. 11 shows an exemplary display of the histogram image according to a fifth embodiment. In the embodiment, the histogram with the arbitrary elastic modulus is designated among those shown in the first embodiment so as to display the specific histogram information. Specifically, plural cursors (C1, C2, ...) are displayed on the histogram image data via the input means of the system control interface unit 17 so as to allow the operator to freely move the cursors. The histogram information at the cursor C1, C2, ... designated by the operator, for example, numeric data such as the number of counts N1, N2,. the average value m1, m2 may be displayed in synchronization with the cursor movement.

The embodiment further allows the operator to evaluate the binding of the target region with the peripheral tissue in terms of the rigidity in the objective and quantitative manner, and makes sure to assist in the tissue identification.

Sixth Embodiment

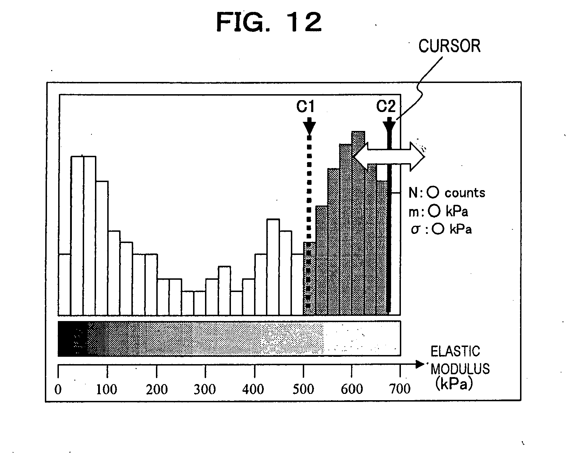

Fig. 12 shows an exemplary display of the histogram image according to a sixth embodiment. In the embodiment, the histogram range is designated by the cursors C1, C2 ... shown in a fifth embodiment to calculate the statistic property in the range and the resultant image is displayed.

In the case where the range is defined on the histogram by the cursors C1 and C2 as shown in Fig. 12, the statistic properties such as the number N of the measurement points, the average value m, and the standard deviation σ of the histogram data corresponding to the range are calculated to be displayed as the numeric data.

The range on the histogram arbitrarily designated by the operator using the cursors C1 and C2 is only subjected to the fitting with the approximate function as described in the fourth embodiment such that the statistic properties are extracted to be displayed as the image.

Seventh Embodiment

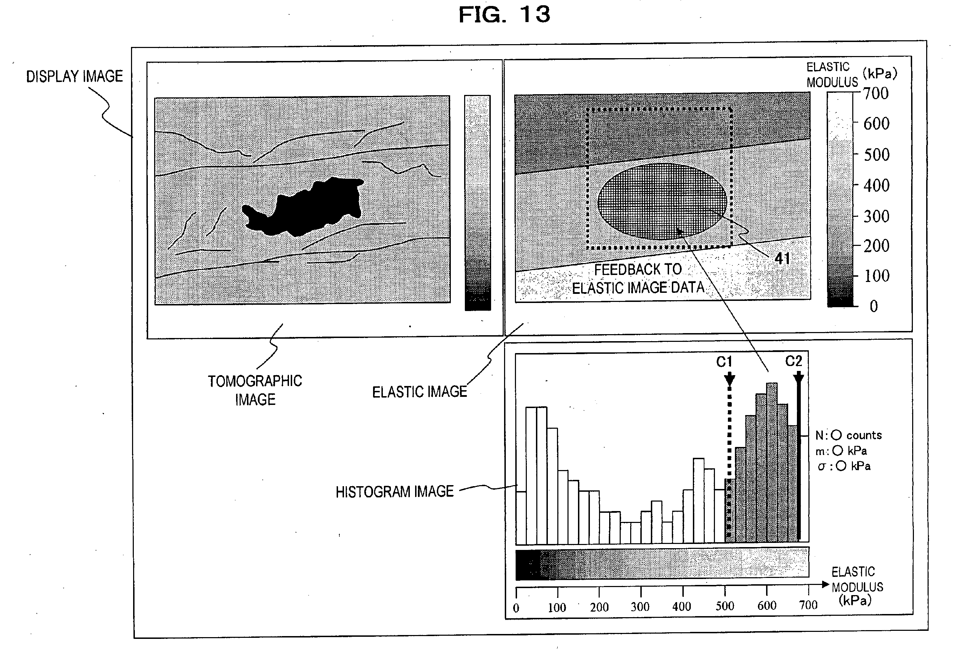

Fig. 13 shows an exemplary display of the histogram image according to a seventh embodiment. The embodiment is structured to display the region of the elastic image corresponding to the block of the measurement points in the region of the selected histogram between the cursors C1 and C2 in the sixth embodiment, which is shown as a shaded region 41 of Fig. 13. The operator is allowed to easily recognize the correlation between the histogram and the tissue.

Eighth Embodiment

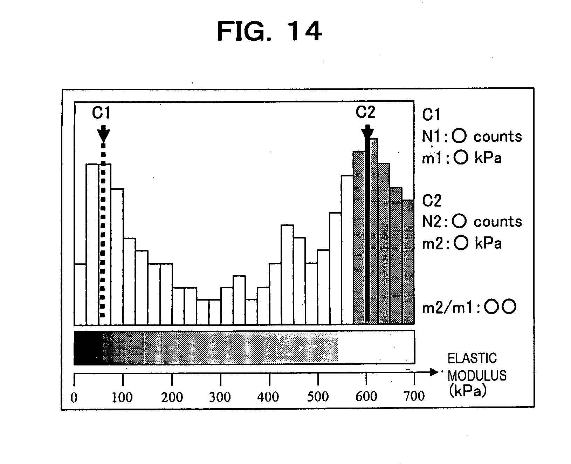

Fig. 14 shows an exemplary display of the histogram image according to an eighth embodiment. The embodiment displays the calculated ratio of the elastic modulus at two peak positions C1 and C2 on the histogram, that is, m2/m1. Assuming that the C1 is the region of the normal tissue, the embodiment allows the operator to recognize as to how many times the rigidity of the tissue in the region C2 is higher than the C1 immediately.

Conventionally, the body tissue is pressed such that the resultant distortion and the elastic modulus in the respective inner region are calculated, and elastic image developed by grading with the level is displayed. However, it is impossible to allow the operator to recognize how the target region is bound with the peripheral tissue in terms of the rigidity. Meanwhile, the present invention allows the operator to evaluate the binding of the target region with the peripheral tissue in terms of the rigidity in the objective and quantitative manner and'makes sure to perform the tissue identification.

There has been often the case where the diagnosis is made based on the level of the brightness (hue) and the extent thereof on the elastic image which may be subjectively determined by the operator, resulting in variance in the diagnostic results depending on the operators. The embodiment avoids the subjective determination to make sure to provide the objective and universal diagnosis and the clinically useful ultrasonic diagnostic system.

Ninth Embodiment

The embodiment adds the following function to the histogram evaluation unit 20 in addition to the eighth embodiment.

Each half-width of the elastic modulus of the peak portion designated by the cursors C1 and C2 of Fig. 14 is obtained, based on which the tissue property is evaluated. The evaluation result is output and displayed. For example, if the half-width of the elastic modulus is in the range from 1 to 20 kPa, it is determined as the cancer tumor which is the considerably rigid solid tissue. If the half-width of the elastic modulus is in the range from 21 to 70 kPa, it is determined as the fiber adenoma which is the flexible solid tissue. If the half-width of the elastic modulus is 71 kPa or larger, it is determined as the cyst which is the mobile cystic tissue.

The ratio between the variance/average at the peak portion is obtained and formed to be non-dimensional. In case of the cancer tumor, the variance is small and the average is large, and accordingly, the ratio therebetween becomes small (for example, 0.1). In case of the fiber adenoma, the variance is the intermediate value, and the average is also the intermediate value. Accordingly, the ratio between the variance and the average becomes intermediate (for example, 0.3). In case of the cyst, the variance is large, and the average is small. The resultant ratio between the variance and the average becomes large (for example, 0.5) .

The embodiment is capable of outputting and displaying the evaluation results of the tissue property, thus further improving the reliability in the tissue identification.

Tenth Embodiment

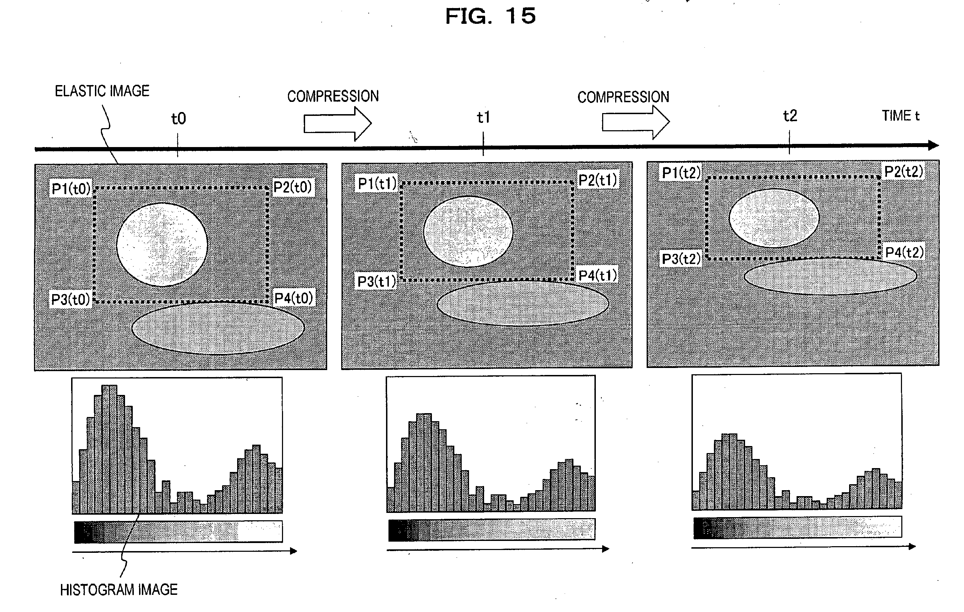

Fig. 15 is an exemplary display of the histogram image according to a tenth embodiment. In the embodiment, the ROI set on the elastic image is automatically changed by following the movement of the tissue which displaces accompanied with the pressure, and the histogram in the thus changed interest region is displayed correspondingly. As the ROI is set by tracking the same tissue region, the appropriate histogram information may be obtained.

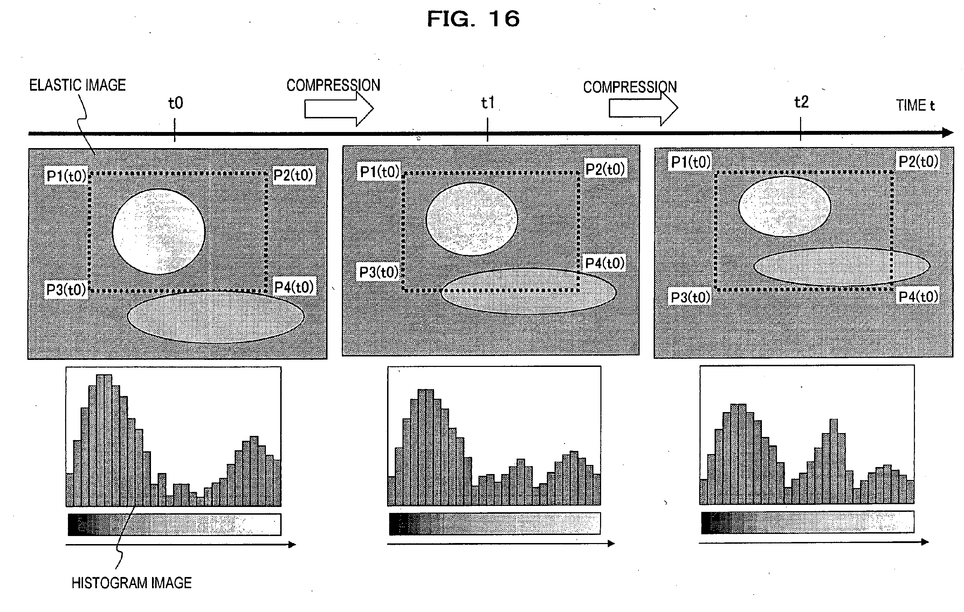

Generally the elasticity may be formed into the image using the ultrasonic wave as the elastic image calculated during the compression to the subject by manually moving up and down the ultrasonic probe in contact with the body surface of the subject at the diagnostic point. If the set ROI coordinate data are kept fixed as shown in Fig. 16, the tissue which displaces with the pressure deviates from the ROI, or the tissue outside the ROI may enter into the ROI, thus changing the population in the ROI. Calculation of the histogram of the measurement points in the ROI may not allow the objective comparison. Accordingly, the comparison with the elastic images obtained in the state where the compression force changes as time elapses may fail to provide the appropriate identification information.

In the embodiment, the coordinate data of the ROI are updated in response to the compression state of the subject, and the state of the body in motion such as the pulsating. More specifically, as shown in Fig. 6 the histogram evaluation unit 20 receives the points which represent the ROI region set by the system control interface unit 17, for example, P1 (t0) (x1,y1), P2 (t0) (x2, y1), P3 (t0) (x1, y2), and P4 (t0) (x2, y2) at ROI setting time point, t0. Then at a time point t1, the displacement frame data $D_{i, j}$ (t1) at the time point t1 are input from the displacement measurement unit 12 as shown in Fig. 1. In reference to the displacement data of the coordinate corresponding to the displacement frame data $D_{i,j}$(t1) obtained at the time point t1, the position of the tissue at the time point t1 displaced from the ROI with the coordinates of P1 (t0) (x1,y1), P2 (t0) (x2,y1), P3(t0)(x1,y2) and P4(t0)(x2,y2) at the time point t0 may be obtained. The obtained coordinates P1(t1), P2(t1), P3(t1) and P4(t1) are updated as the new ROI coordinate data. The histogram and the statistic properties are obtained using the population which distributes in the updated RQI coordinate so as to be displayed.

In the embodiment, the body is compressed to bring the tissue inside the body close to the ultrasonic probe during the compression. The processing of the embodiment is repeated at the arbitrary time point to update the ROI coordinate data in response to the compression state of the subject, the state of the body in motion such as the pulsating. In the embodiment, the position and the size of the ROI may be changed depending on the time as shown in Fig. 15. The same tissue region in the body may be extracted at the arbitrary time point.

In the embodiment, the population is formed based on the elastic frame data corresponding to the same tissue region so as to calculate the histogram and the statistic properties to be displayed. This may prevent the other tissue from entering into the ROI during the compression stage. As a result, the number of the samples in the population is changed. However, statistic property such as the average and the variance as the important information for differentiating the tissue feature may be maintained as the stable value at the arbitrary time. This makes sure to evaluate the value inherent to the tissue.

Referring to Fig. 16, if the ROI coordinates are fixed, the calculated histograms vary depending on the time. The indexes representing the statistic properties calculated based on the histogram may also vary depending on the time. The aforementioned process may interfere with the objective and reliable diagnosis. In the embodiment, the ROI coordinate data are updated in response to the compression process to extract the same tissue region at the arbitrary time point, and to evaluate the resultant statistic properties. This makes it possible to make the diagnosis in the objective and reliable manner with high accuracy.

Eleventh Embodiment

An eleventh embodiment shows another exemplary display of the histogram image. In the embodiment, the histogram evaluation unit 20 stores the previously obtained histogram data to form cumulative histogram data by temporally accumulating the histogram data of the respective pins at the current time, and the previous histogram data. The statistic property of the cumulative histogram data may be obtained such that the image is displayed.

The histogram evaluation unit 20 may be structured to store the previously obtained histogram data to form smoothing histogram data by temporally smoothing the histogram data for the respective pins at the current time and the previous histogram data. In this case, the statistic property of the smoothing histogram data may be obtained such that the image thereof is displayed. The embodiment may extract the stable and accurate static' property amount.

Twelfth Embodiment

The aforementioned respective embodiments show examples where the histogram and the statistic property are calculated in real time so as to be displayed. However, the present invention is not limited to the aforementioned structures. The image data stored in the cine-memory unit 18 may be used to perform the required processing as shown in Fig. 2.

The aforementioned process is performed while freezing the system via the system control interface unit 17. The cine-memory unit 18 calls the previous display image data in accordance with the control signal of the system control interface unit 17 so as to designate the display image data and the ROI for evaluation with the histogram among those of the arbitrary frame. The coordinate data of the ROI are output to the histogram evaluation unit 20. The information which designates the frame to be processed is output to the distortion/elastic modulus calculation unit 13 such that the previous elastic frame data stored therein are read and transmitted to the histogram evaluation unit 20 for calculating the histogram and the statistic property. This makes it possible to allow the present invention to be implemented off-line.

Thirteenth Embodiment

The determination whether or not the histogram is displayed as described in the aforementioned embodiments may be arbitrarily made, or the display of the histogram (pin width, each range of horizontal axis and vertical axis) may be arbitrarily set or changed. The determination as to which statistic information including the average value, standard deviation of the statistic property is calculated and displayed may be arbitrarily made by the system control interface unit 17.

Fourteenth Embodiment

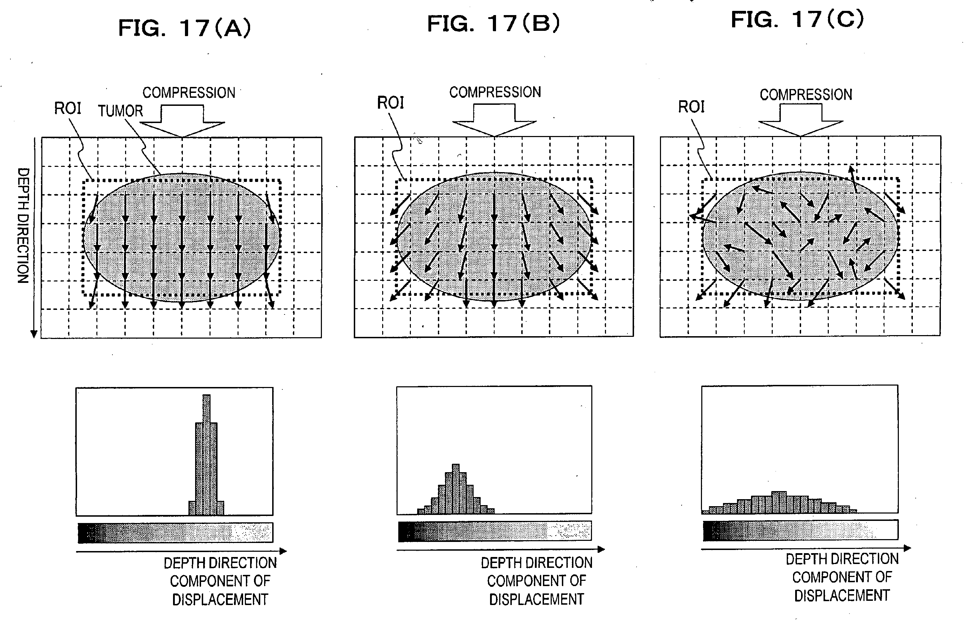

Fig. 17 shows an exemplary display where the histogram is generated using the displacement vector as one of the elastic data correlating with the elasticity according to a fourteenth embodiment of the present invention. In the embodiment, based on the ultrasonic tomographic data measured by pressing the tissue of the subject body, the displacement vector of the tissue at the plural measurement points in the tomographic site of the subject is obtained to generate the elastic image showing the displacement vector corresponding to the measurement point in the tomographic site. Then the distribution of the displacement vector component in the depth direction as the specific direction in the interest region to be set for the elastic image is displayed in the histogram. The horizontal axis of the histogram in the drawing denotes the depth direction component of the displacement.

Fig. 17(A) shows that the target region set in the ROI is the considerably rigid solid tissue as the cancer tumor. As arrows in the upper view of Fig. 17(A) show, each displacement vector at the respective measurement points is directed in the same direction. The histogram showing the distribution of the displacement vector in the ROI becomes the one concentrated in the narrow range as shown in the lower view of Fig. 17(A). As the tissue elements are bound strongly, they may be in the motion state without deforming the entire structure, even if it is pressed.

Fig. 17(B) shows that the target region set in the ROI is the flexible solid tissue as the fiber adenoma. The displacement vectors of the respective measurement points are directed in relatively wider distributed directions as shown with arrows in the upper view of Fig. 17(B). The distribution width of the histogram showing the displacement vectors in the ROI is relatively widened as the lower view of Fig. 17(B) shows. The histogram shows that the tissue elements are bound to a certain degree so as to allow the deformation a certain degree upon reception of the pressure while binding with one another.

Fig. 17(C) shows that the target region set in the ROI is the mobile cystic tissue as the cyst. The displacement vectors at the respective measurement points are directed in various directions as shown with arrows in the upper view of Fig. 17(C). The distribution width on the histogram showing the displacement vectors in the ROI is widened as the lower view of Fig. 17(C) shows. The histogram shows that the tissue elements are not bound with one another such that the respective elements move in the arbitrary directions upon reception of the pressure.

It is to be understood that the difference in the tissue property may be identified by forming the histogram by the component of the depth direction of the displacement vector or the component of the lateral direction. Besides the difference in the rigidity, the binding level of the tissue elements may be identified by observing the distribution width. The effective auxiliary elastic information for identifying the tissue may be obtained. Fifteenth Embodiment

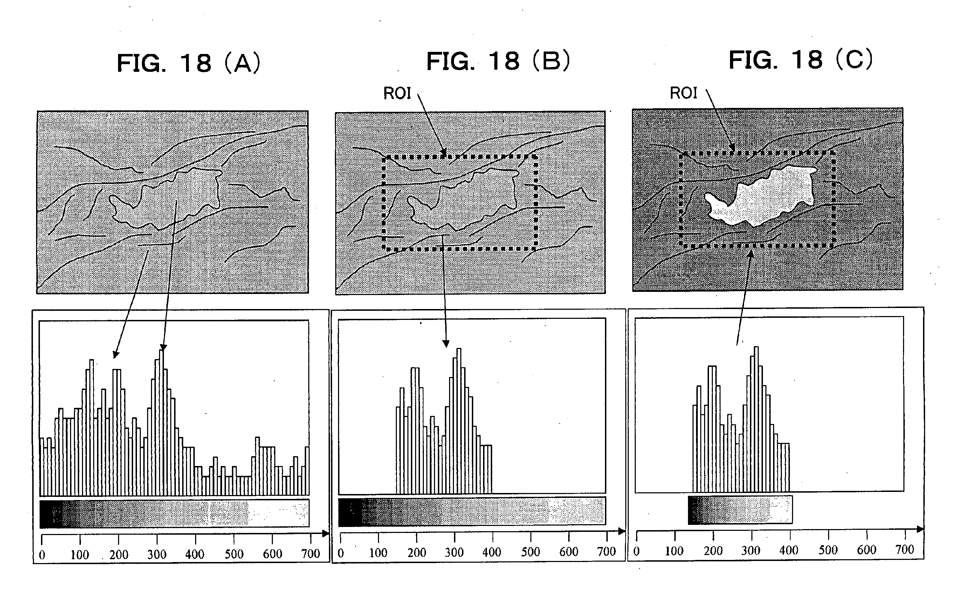

Fig. 18 shows an exemplary display of the elastic information according to a fifteenth embodiment of the present invention. In the embodiment, the range for allocating the gradient (256 gradients) with the hue or brightness of the elastic image is changed in accordance with the width of the elastic data of the histogram in the interest region ROI to emphasize the contrast of the difference in the elasticity within the interest region ROI. Fig. 18(a) shows the elastic image and the histogram in the field of view. Fig. 18(b) shows the histogram at the measurement points in the interest region ROI which has been manually or automatically set by detecting the boundary in the elastic image. Fig. 18(c) shows the allocation range of the gradient automatically changed in accordance with the width of the elastic data of the histogram at the measurement points in the interest region ROI to display the emphasize contrast of the difference in the elasticity within the ROI.

The embodiment displays the distribution of the rigidity of the tissue in the ROI while emphasizing the contrast to provide effective auxiliary elastic information for identifying the tissue.

Sixteenth Embodiment

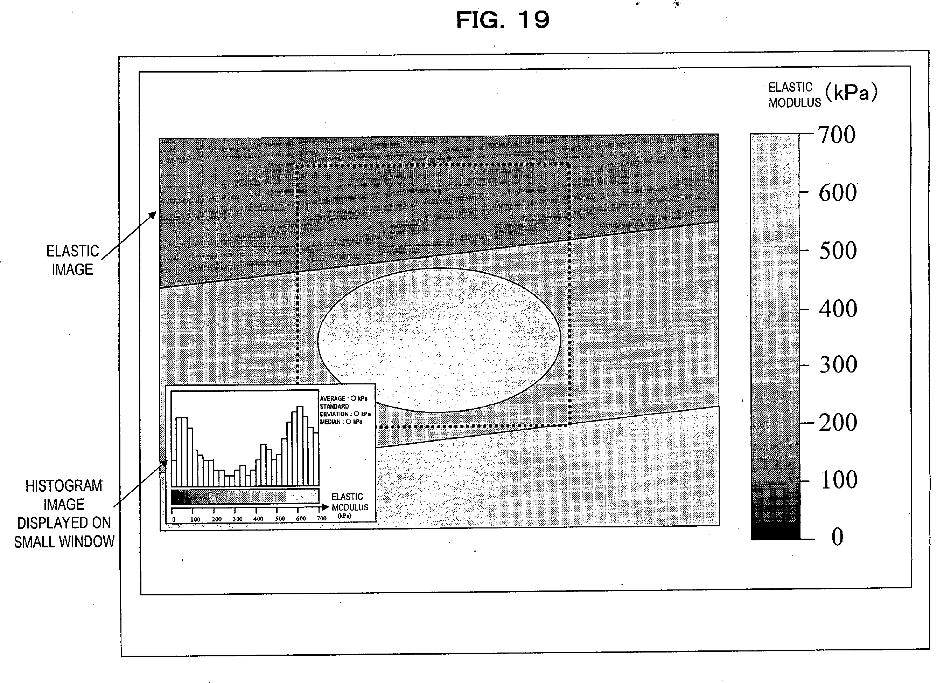

Fig. 19 shows an exemplary display of the elastic information according to a sixteenth embodiment of the present invention. In the exemplary display of the elastic information according to the first embodiment as shown in Fig. 9, the auxiliary elastic information image which includes the elastic image and the histogram (histogram image) is displayed on the single screen. In the embodiment, the histogram image is displayed on the small window so as to be partially superimposed on the elastic image as shown in Fig. 19.

The histogram image may be displayed at the sacrifice of the resolution because it is in no danger of the oversight problem. Accordingly, the histogram image does not have to be displayed in the same size as those of the tomographic image and the elastic image. As shown in the embodiment the histogram image having its size reduced may be displayed on the small window in the image region of the elastic image. As the operator is allowed to observe the elastic information of the elastic image at the sufficient resolution, the information loss may be suppressed.

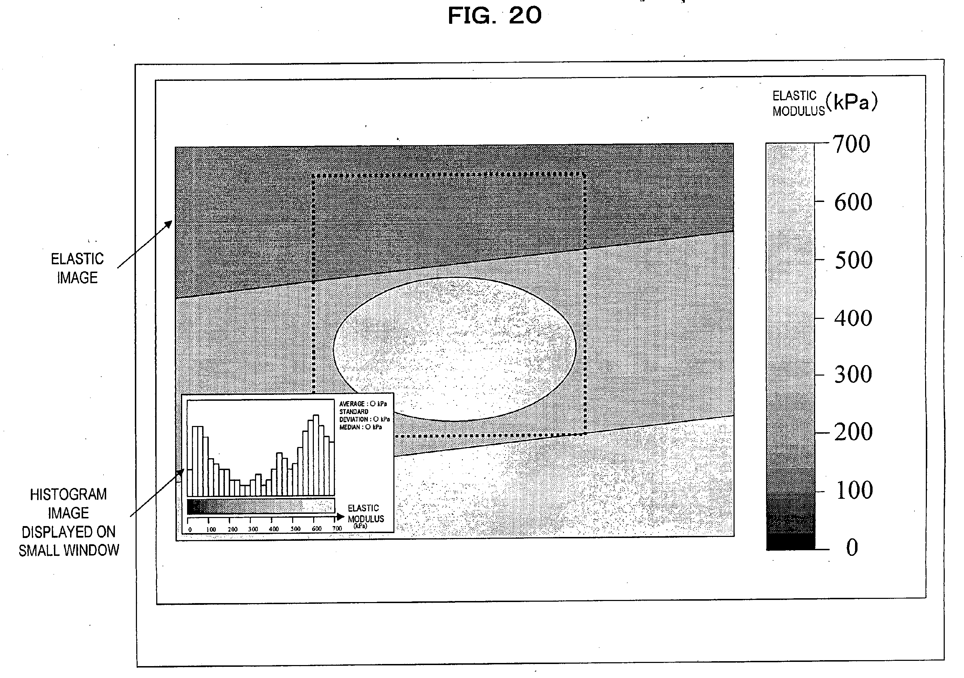

In the example of Fig. 19, as the histogram image is partially superimposed onto the elastic image to hide the' information contained therein, the operator may miss the important elastic information as the case may be. To avoid the aforementioned problem, it is preferable to form the histogram image to be half-transparent and superimposed onto the elastic image as shown in Fig. 20. Accordingly, missing of the elastic information hidden by the histogram image superimposed thereto may be prevented.

The generally employed image development process so called alpha blending for generating the colored half-transparent image will be described. Assuming that the color information of the pixel is expressed by three primary colors (R, G, B), the color information $E(i,j)$ of the element in the coordinate $(i,j)$ of the colored elastic image becomes the function of the color information of the three primary colors, that is, $Er(i,j)$, $Eg(i,j)$ and $Eb(i,j)$ as expressed by the following equation.

$$E(i,j) = (Er(i,j), Eg(i,j), Eb(i,j)).$$

Likewise as above, the color information H(i,j) of the pixel at the colored histogram image at the coordinate (i, j) becomes the function as follows.

$$H(i,j) = (Hr(i,j), Hg(i,j), Hb(i,j)).$$

Assuming that the superposition ratio between both images is set to $\alpha$ ($0 \le \alpha \le 1$), the color information G (i, j) of the pixel of the half-transparent superimposed image at the coordinate (i,j) in the region where the histogram image is superimposed may be obtained by the following equation:

$$C(i,j) = \alpha \times E(i,j) + (1-\alpha) \times H(i,j).$$

The color information of the pixel of the half-transparent superimposed image at the coordinate (i,j) includes the image information of both the elastic image and the histogram image at the ratio of $\alpha$, which allows the respective color information data to be simultaneously recognized through the half-transparent views.

As mentioned above, the embodiment prevents deterioration in the accuracy of the image diagnosis, if each of the plural images is reduced into small but the same size to be disposed on the limited screen of the single monitor simultaneously so as not to interfere with one another.

Seventeenth Embodiment

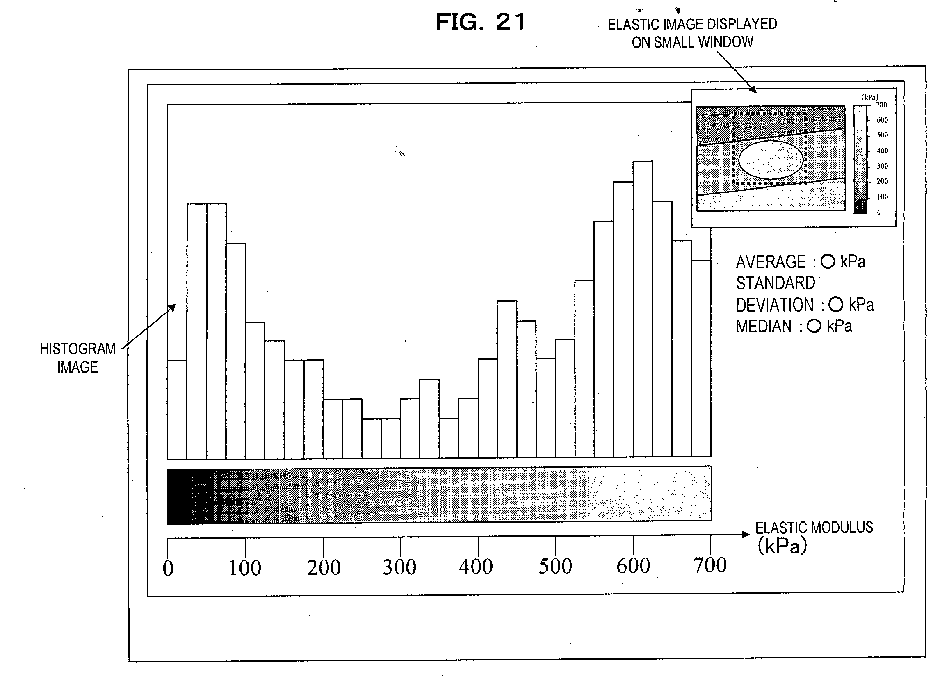

Fig. 21 shows an exemplary display of the elastic information according to a seventeenth embodiment. Figs. 19 and 20 show that the contracted histogram image is displayed on the window in the region of the diagnostic image such as the tomographic image and the elastic image. In the embodiment, the relationship of the diagnostic image and the histogram image with respect to the size on the display region may be inverted in need as shown in Fig. 21.

The embodiment may be structured to switch the relationship of size between the diagnostic image and the histogram image on the display region by operating the image selection key provided on the predetermined key or button of the system control interface unit 17 in the ultrasonic diagnostic system. Besides the key and the button of the system control interface unit 17, the image selector key may be formed as an icon displayed on the monitor image.

The embodiment.allows the cursor movement on the histogram image to be efficiently performed on the enlarged histogram image in the embodiment as shown in Figs. 11 and 12.

Eighteenth Embodiment

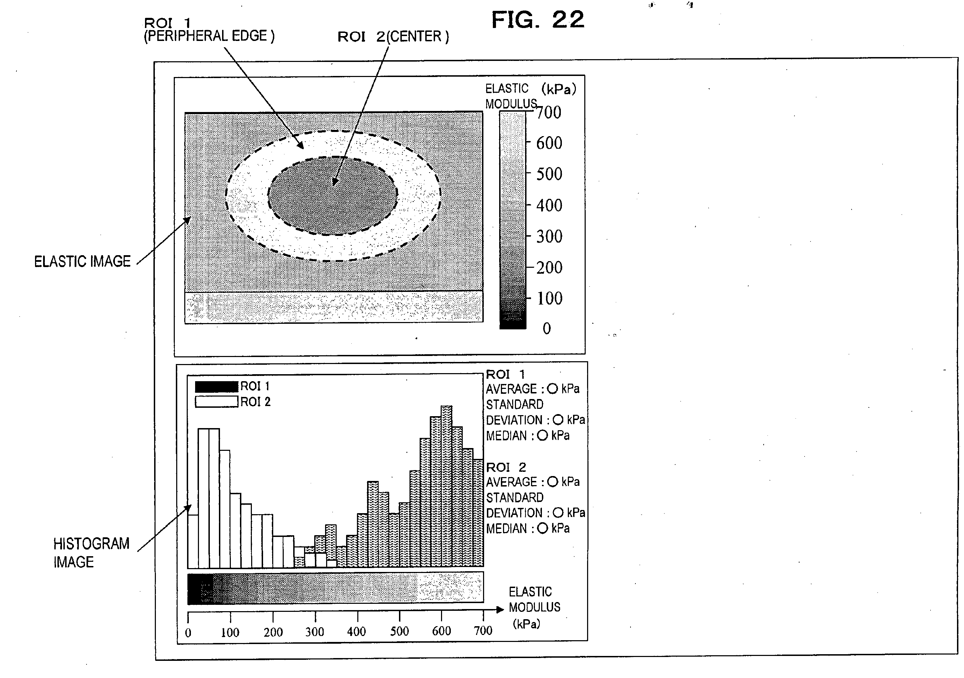

Fig. 22 shows an exemplary display of the elastic information in the case where the present invention is applied to the diagnosis for identifying the cystic cancer in the thyroid gland. Identification of the cystic cancer in the thyroid gland is considerably important for the image diagnosis with respect to the thyroid gland. It is well known that the cystic cancer has the cell density which distributes inside the tumor. The peripheral edge of the tumor has the cell density higher than that of the center of the tumor. In the case where the cystic cancer is evaluated in reference to the elastic image, the peripheral edge of the tumor is likely to be displayed as the image indicating the rigid region compared with the center of the tumor. In other words, the site with the higher cell density tends to be measured as being rigid compared with the site with the lower cell density.

The embodiment displays the appropriate elastic information for evaluating the feature of the elasticity distribution inside the tumor. For example, there may be the case where the difference in the rigidity between the peripheral edge and the center of the tumor may not be recognized by the generally employed elastic image especially in case of the tumor in the thyroid gland. Fig. 22 shows an exemplary display of both the elastic image of the thyroid gland tumor and the histogram image in case of the cystic cancer. Referring to the drawing, the histogram is analyzed regarding the center of the tumor ROI2 and the annular peripheral edge of the tumor ROI1 as the independent populations, to obtain and display effective information for identifying the cystic cancer objectively.

In the embodiment, the images are displayed as follows.

(1) The peripheral edge and the center of the region doubted as the cystic cancer are set to ROI1 and ROI2, respectively so as to be displayed.
(2) Each histogram of the ROI1 and ROI2 is discriminated by different colors, and synthesized histogram image is displayed.
(3) Each statistic information of the histograms is calculated and displayed. In the drawing, the average, the standard deviation and the median are displayed.

The ROI1 and ROI2 may be set through various processes. For example, the operator is allowed to designate the outer periphery and the inner periphery of the annular peripheral edge ROI1 by drawing the lines using the input means such as a mouse and a track ball of the system control interface 17. The annular region between the drawn lines is recognized as the peripheral edge ROI1 to be set.

The operator is allowed to adjust two oval templates selected from the plural preliminarily prepared oval templates to the outer and the inner peripheries of the peripheral edge ROI1 so as to be set by operating the mouse and the track ball. The annular region defined by the outer peripheral lines of the two oval templates is recognized to be set as the peripheral edge ROI1, and the inner region of the small oval template is recognized to be set as the ROI2.

The operator is allowed to dispose two torus (annular) ROIs on the image among the previously prepared plural torus ROIs suitable for designating the peripheral edge by operating the mouse and the track ball. The annular region defined by the two torus ROIs is considered as the peripheral edge ROI1, and the inner region of the small torus ROI is considered as the ROI2 so as to be set.

The embodiment allows the quantitative and objective diagnosis with respect to the cystic cancer in case of the use of the elastic image which cannot be recognized with respect to the difference in the rigidity between the peripheral edge and the center of the tumor quantitatively. Nineteenth Embodiment

Generally, the inspection so called the dynamic test to evaluate the movability (mobility) of the tumor for performing the image diagnosis on the mammary gland region has been well known as introduced in the reference titled "Dynamic tests in real-time breast echography" by Ueno E, et al, Ultrasound Med Biol., 14: 53-57, 1988. The dynamic test is intended to evaluate the level how the interest tumor is bound with the peripheral tissue. More specifically, the diagnosis is made to evaluate whether the target tumor is moved accompanied with the movement of the peripheral tissue or it moves independently without being influenced by the movement of the peripheral tissue when the entire breast is largely deformed while observing the tomographic image in real time. For example, as the madidans tumor of the breast cancer grows wet in the peripheral tissue, it is highly bound with the peripheral tissue with the low mobility. Meanwhile, as the benign tumor shows no madidans property, its movement is independent from that of the peripheral tissue. The mobility of the benign tumor has high mobility. However, as the evaluation on the mobility depends on the experience of the operator, which is likely to be made subjectively, the resultant evaluation is less objective.

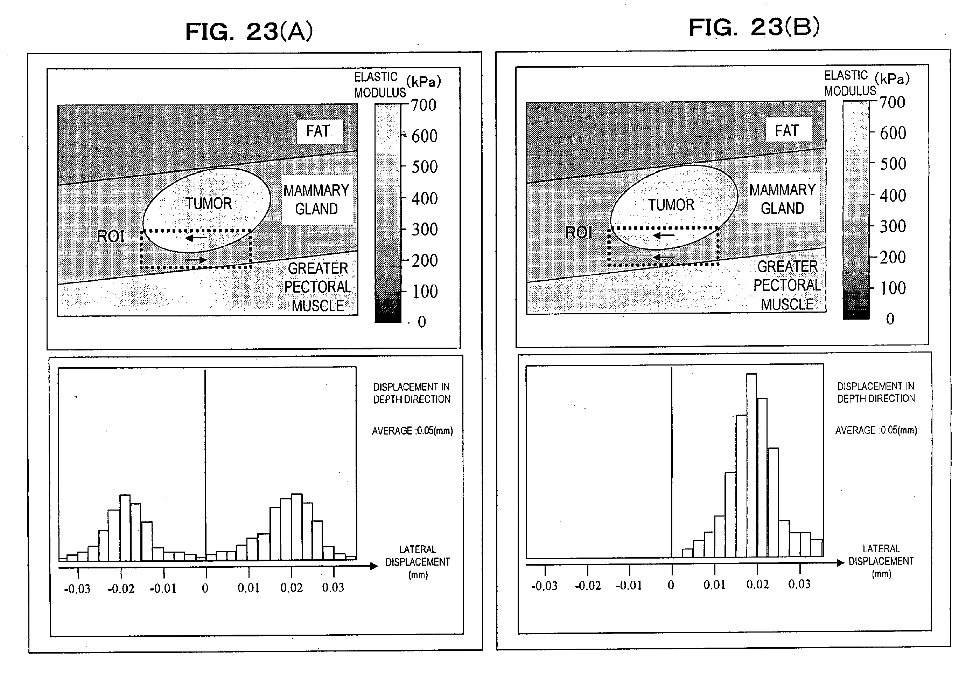

In the embodiment, the mobility is evaluated using the histogram. Figs. 23(a) and 23(b) show exemplary displays of the elastic information according to the embodiment. The interest site shown in Fig. 23(a) is expected to be the tumor with high mobility such as the benign tumor which is not bound with the peripheral mammary gland. When the breast is largely deformed from outside, the tumor slips at the boundary with the mammary gland. That is, there are differences in the displacement direction and displacement amount between the boundary region at the tumor side and the boundary region at the mammary gland side. The displacement may be made in the opposite directions as the case may be. Referring to Fig. 23(a), the ROI is set in the region which contains the boundary between the tumor and the mammary gland. Then the compression operation applied when the displacement of 0.05 mm is made in the depth direction in the ROI is set as the criterion of the evaluation. When the amount of the lateral displacement in the ROI is displayed into the histogram, there are two peaks in the histogram each having the same amount of the displacement in the positive direction (rightward on the image) and in the negative direction (leftward on the image). The extent of the histogram (half-width of the peak, standard deviation, variance) and the distance between the peaks (mm) may indicate the level of the mobility so as to be used for the evaluation.

Meanwhile, the interest site shown in Fig. 23(b) is assumed to be the malignant tumor with low mobility. As the tumor is strongly bound with the peripheral mammary gland, the tumor and the mammary gland around the boundary take the similar motions when the entire breast is largely deformed outside. The differences in the displacement direction and the displacement amount between the boundary region at the tumor and the boundary region at the mammary gland around the boundary are small. The tumor and the mammary gland may be completely bound and displace in unison as the case may be. Likewise the case as shown in Fig. 23(a), the ROI is set on the region including the boundary between the tumor and the mammary gland. The compression applied when the displacement of 0.05 mm in the depth direction is made in the ROI is set as the criterion of the evaluation. The histogram which represents the amount of the lateral displacement in the ROI is shown in Fig. 23(b). The peak of the histogram with the similar displacement amount occurs in one of the positive direction (for example, rightward on the image) or the negative direction (for example, leftward on the image). The extent of the histogram (half width of the peak, standard deviation, and variance) may indicate the level of the mobility so as to be used for the evaluation.

Twentieth Embodiment

In the nineteenth embodiment, the histogram based on the displacement information is provided as the auxiliary elastic information for objectively evaluating the mobility which relates to the humidity coefficient of the tumor to its periphery as one of the information data to determine whether the tumor is benign or malignant. For the aforementioned object in the embodiment, the method which employs the histogram will be described as the auxiliary elastic information for evaluating the syncretio between the tissues.

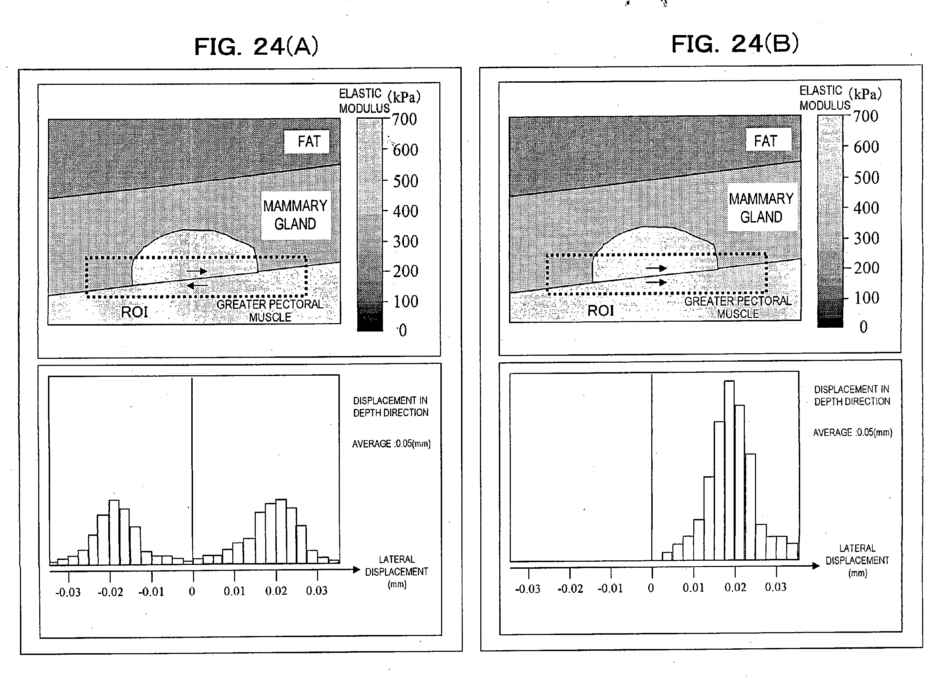

Fig. 24 shows an exemplary elastic images in the case where the tumor exists in the mammary gland tissue. The drawing shows the auxiliary elastic information suitable for the operation to extract the tumor around the boundary between the mammary gland and the greater pectoral muscle. When the tumor around the boundary between the mammary gland and the greater pectoral muscle is extracted, the approach to the tumor is made from the intermediate portion between the mammary gland and the pectoral muscle. In the aforementioned operation, the determination whether or not the tumor and the greater pectoral muscle, and the mammary gland and the greater pectoral muscle are separable without the syncretio becomes considerably important for the operation.

In the embodiment, the ROI is set in the region required to be evaluated with respect to the syncretio between the mammary gland and the greater pectoral muscle as shown in Fig. 24(a). Likewise the case as shown in Fig. 23, if there is no syncretio between the mammary gland and the greater pectoral muscle, they will move independently in response to the deformation of the breast. In this case, the slippage occurs at the boundary. In the case where the predetermined amount of the displacement in the depth direction is made to display the histogram images for indicating the lateral displacement, two peak portions appear in the histogram as shown in Fig. 24(a).

Meanwhile, if the strong syncretio occurs between the mammary gland and the greater pectoral muscle strongly, the mammary gland is bound with the greater pectoral muscle to move in unison as shown in Fig. 24(b). Accordingly, the distribution having the peak at the constant displacement is obtained. The extent of the histogram in the ROI (half-width of the peak, standard deviation, and variance) may be the criterion for evaluating the possibility of the syncretio or separation.

The histogram based on the displacement information may be used for evaluation to determine with respect to the possibility of separation as the important information for planning the operation to extract the tumor.

Besides the extraction operation, the evaluation with respect to the syncretio between tissues may be effectively used for the breast implant by inserting the bag (saline bag or silicon bag) between the mammary gland and the greater pectoral muscle. The similar effect may be derived from the application to the surgery using the endoscope.

Twenty-first Embodiment

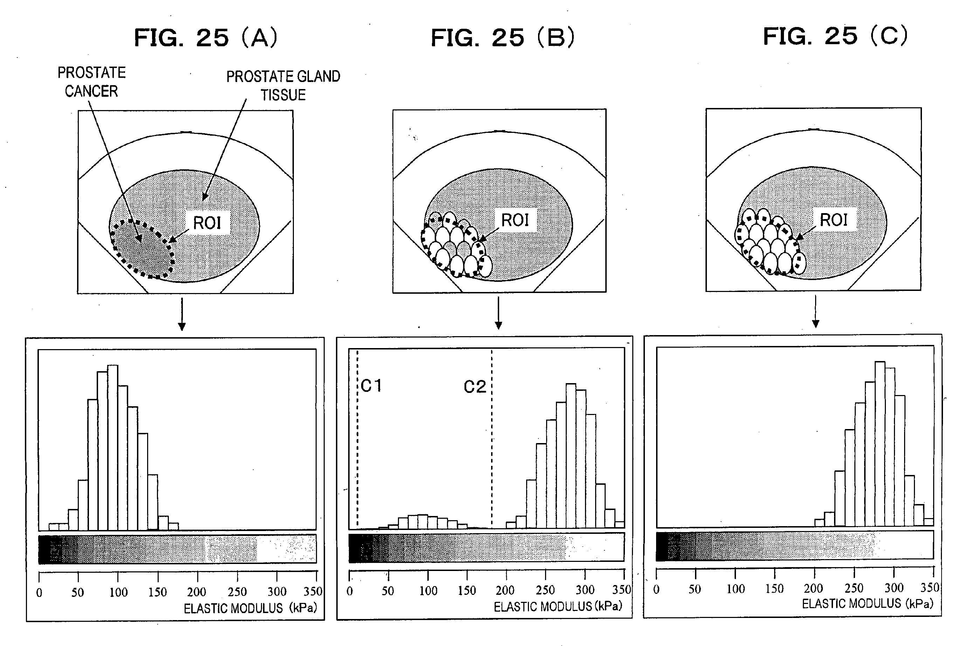

Referring to Fig. 25, a twenty-first embodiment shows the application of the quantitative auxiliary elastic information of the present invention to the determination with respect to the treatment effect of the high-intensity focused ultrasound (hereinafter referred to as HIFU).

The HIFU treatment is performed to destroy the tissue of the diseased site (for example, lesion such as cancer) by irradiating the ultrasound to the site to be treated inside the subject body from the ultrasonic treatment probe, focusing the high-intensity ultrasound to the small region of the site to be treated, and heating the focal site to a high temperature ranging from 60 to 90°C. The treatment is performed by destroying the entire tissue of the diseased site while gradually moving the focal point in the site to be treated.

As the tissue of the diseased site destroyed by the treatment becomes rigid through the thermal denaturalization. The level of the rigidity, thus may be correlated with the treatment effect. The determination whether or not the treatment has been uniformly performed over the entire tissue of the site to be treated may be made by observing the elastic image which represents the elastic information measured in the interest region ROI at the site to be treated. The elastic image displayed in the gradient corresponding to the rigidity fails to identify the gradient difference in the considerably tiny site in the region where the color gradients are approximated as described in Background Art. Accordingly, there may be the case where the determination whether or not the treatment has been performed cannot be clearly made. In this case, the histogram as the auxiliary elastic information according to the present invention may be used for assisting in the appropriate diagnosis in the quantitative and objective manner.

Fig. 25(a) shows the elastic image each representing the cross-sectional view of the prostate gland including the prostate cancer before the treatment. As the ROI is set in the region of the prostate gland cancer, the image shows the elasticity of approximately 100 (kPa) as shown in the drawing.

Fig. 25(b) shows the intermediate stage of the HIFU treatment applied to the region of the prostate cancer. The area of the prostate cancer sufficiently radiated with the ultrasound becomes rigid through the thermal denaturalization. The histogram shows the change in the rigidity to approximately 300 (kPa) as shown in Fig. 25(b). As the histogram in Fig. 25(b) shows, the tissue region with the rigidity of 100 (kPa) indicates that the untreated prostate cancer is left in the ROI. The aforementioned regions denote the one where the treatment has not been completed yet owing to insufficient irradiation of the ultrasound.

Likewise the embodiment shown in Fig. 13, the incomplete treatment region is selected by the cursors C1 and C2, and the measurement points are feedbacked to the elastic image to be displayed. This makes it possible to display the incomplete treatment region on the image to be recognizable by the operator.

On the final step, the HIFU treatment is applied again to the incomplete treatment region with insufficient thermal denaturalization as shown in Fig. 25(c). This makes it possible to confirm that the treatment to the entire region in the ROI has been uniformly applied in reference to the histogram.

The threshold value of 200 (kPa) or higher may be set as the criterion for determination with respect to completion of the treatment. The common condition may be set for determining the effect of the treatment to make sure the appropriate treatment to be performed in accordance with the constant treatment criterion.

The treatment method by burning the cancer tissue with heat is not limited to the use of HIFU, but RFA (percutaneous radiofrequency wave ablation treatment) may be employed. The present invention may also be applied to cryo treatment by sharply cooling the cancer tissue in view of hardening through the thermal denaturalization.

The present invention is applicable to the method for evaluation whether or not the treatment has been uniformly performed, or the treatment effect has been uniformly obtained with respect to the treatment for changing the rigidity owing to the tissue denaturalization, which is applied to the cancer tissue as well as the hormonal therapy with the medication.

Twenty-second Embodiment

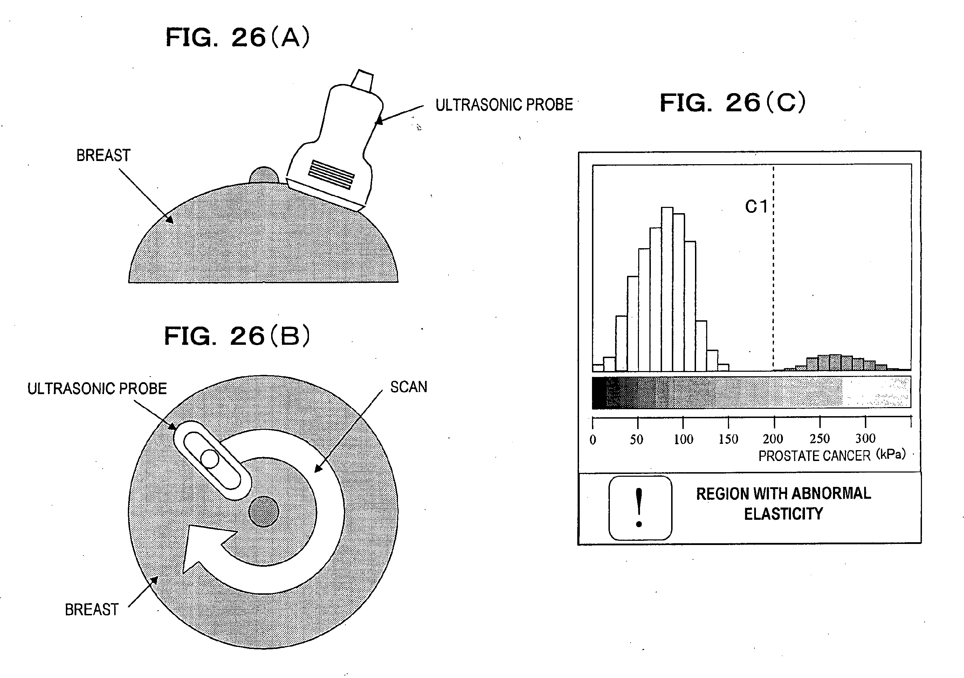

Referring to Fig. 26, a twenty-second embodiment of the present invention suitable for the breast cancer screening will be described. In the process for screening the breast cancer, the ultrasonic probe is brought into contact with the breast to scan the tomographic image of the breast in real time as shown in Figs. 26(a) and 26(b). In the aforementioned screening, the time for screening of the subject is limited, and accordingly, efficient checking is required. The observation of all the regions on the measurement cross section displayed as the tomographic image which is scanned in real time without the oversight problem requires much time, and depends largely on the experience. The tomographic image such as the B mode image indicates the information of the structure of the tissue in the body developed as the image, and may be used for identifying the information of the mode of the tissue, for example, whether the tumor image is formed, the rear echo is intensified, the peripheral edge portion is roughened, and the like. The experience of the operator is required for the efficient diagnosis as the tumor is detected or identified based on the large number of complex views.

As the tumor is rigid compared with the peripheral fatty tissue and mammary gland tissue, the observation is made to determine with regard to whether or not the region with the high elastic modulus exists in the measurement cross section upon inspection based on the elastic modulus as the elastic information, for example. The worth the malignant tissue becomes, the higher the rigidity becomes. The determination may only be made whether or not the region with the abnormal rigidity exists in the measurement cross section, thus allowing the operator to recognize it efficiently in real time.

As described in Background Art, however, there may be the case where the difference in the elasticity cannot be clearly discriminated with respect to the boundary region with different color gradients or the region with the approximated gradient of color for identifying the tumor such as the breast cancer based on elasticity difference of the elastic image. The careful observation of all the regions on the measurement cross section based on the elastic images is required. The aforementioned observation while sweeping the measurement cross section in real time may be the burden to the operator.

In the embodiment, the distribution of the elastic information, is formed into the histogram in addition to or in place of the elastic image so as to be displayed as the auxiliary elastic information. This makes it possible to realize the efficient screening. Referring to Fig. 26(c), the elastic information such as distortion or elastic modulus is measured in real time during the scan of the cross-section, and the distribution of the resultant elastic information is formed into the histogram to be displayed. Assuming that the threshold value Eth of the elastic modulus of the tumor doubted to be malignant is set to 200 (kPa), the distribution of the measurement points of the elastic modulus equal to or higher than the value Eth existing on the elastic image may be immediately determined. In other words, the embodiment eliminates the burden for making the confirmation in detail like the case of the elastic image.

The threshold value Cth is set on the vertical axis (level) of the histogram. When the measurement point equal to or higher than Eth is measured to be equal to or higher than the value of Cth, the alarm message of "region with abnormal elasticity" is displayed on the image of the histogram so as to prevent the oversight.

The scale of the vertical axis (level) of the histogram may be changed from the linear scale to the log scale. The log scale makes it possible to detect the very small tumor doubted to be the malignant tumor at lower level with high sensitivity without the oversight.

As described in each embodiment, the auxiliary elastic information which contains the histogram of the elastic distribution in addition to or in place of the elastic image based on the displacement, distortion or the elastic modulus is displayed on the screen of the image display unit 10, thus realizing the efficient diagnosis without giving the burden to the operator.

**Claims**

1. An ultrasonic diagnostic system provided with elastic data calculation means for calculating elastic data of a tissue at plural measurement points of a tomographic site in a body of a subject based on ultrasonic tomographic data measured by exerting a pressure to the tissue of the subject, and elastic image generation means for generating an elastic image of the tomographic site based on the elastic data, comprising unison behavior acquisition means for acquiring a statistic property value representative of a unison behavior of the tissues which form the body of the subject based on the elastic data.

2. The ultrasonic diagnostic system according to Claim 1, wherein the unison behavior acquisition means acquires the unison behavior by forming an elastic distribution based on the elastic data.

3. The ultrasonic diagnostic system according to Claim 1, wherein the unison behavior reflects a property of the tissue.

4. The ultrasonic diagnostic system according to Claim 1, wherein the unison behavior acquisition means includes means for graphing a number of the measurement points with identical or similar elastic data as a histogram while defining the elastic data at all the measurement points contained in an interest region as a population.

5. The ultrasonic diagnostic system according to Claim 4, wherein the unison behavior acquisition means includes means for displaying not only the histogram but also an amount of the property generated by the histogram as auxiliary elastic information data.

6. The ultrasonic diagnostic system according to Claim 1, wherein the unison behavior acquisition means acquires the statistic property amount representative of the unison behavior of the tissue after performing an image processing to the elastic data.

7. The ultrasonic diagnostic system according to Claim 1, further comprising means for allowing the unison behavior acquisition means includes means to evaluate a binding state of a target region of the body of the subject with a peripheral region in terms of rigidity.

8. The ultrasonic diagnostic system according to Claim 1, wherein the unison behavior acquisition means includes histogram evaluation means for generating a histogram representing an elastic distribution on the elastic image, and image display means for displaying at least one of the elastic image and an image of the histogram.

9. The ultrasonic diagnostic system according to Claim 8, wherein the histogram evaluation means generates a histogram which represents the elastic distribution in an interest region set on the elastic image.

10. The ultrasonic diagnostic system according to Claim 8 or 9, wherein the elastic data includes one of a displacement, a distortion and an elastic modulus of the tissue.

11. The ultrasonic diagnostic system according to Claim 8 or 9, wherein the histogram displays numbers of the measurement points with the elastic data corresponding to plural sections formed by dividing the elastic data in lengths.

12. The ultrasonic diagnostic system according to Claim 9, wherein the elastic image generation means automatically changes the interest region set on the elastic image by following the tissue which displaces in accordance with the pressure exerted to the body of the subject.

13. The ultrasonic diagnostic system according to Claim 8 or 9, further comprising means for displaying at least one of an average, a standard deviation, and a median of the elastic data on the histogram together with the histogram image.

14. The ultrasonic diagnostic system according to Claim 8 or 9, wherein the image display means displays a tomographic image of the tomographic site generated based on the ultrasonic tomographic data, the elastic image, and the histogram image on a same screen.

**15.** The ultrasonic diagnostic system according to Claim 8 or 9, further comprising means for displaying at least one of a function approximating the histogram distribution, the elastic data of a peak on the histogram, and a standard deviation of the peak together with the histogram image.

**16.** The ultrasonic diagnostic system according to Claim 11, wherein the histogram evaluation means obtains the number of the measurement points in the section of the elastic data designated by a cursor displayed on the histogram image, and the elastic data in the section, which are displayed in numeric values on the histogram image.

**17.** The ultrasonic diagnostic system according to Claim 11, wherein the histogram evaluation means obtains at least one of the number of the measurement points in plural sections of the elastic data defined by plural cursors displayed on the histogram image, an average value of the elastic data in the plural sections, and a standard deviation of the elastic data in the plural sections so as to be displayed in a numeric value on the histogram image.

**18.** The ultrasonic diagnostic system according to Claim 11, wherein:

the image display means displays a tomographic image in the tomographic site generated based on the ultrasonic tomographic data, the elastic image, and the histogram together on a same screen; and
the histogram evaluation means obtains at least one of the number of the measurement points in the plural sections of the elastic data defined by plural cursors displayed on the histogram image, an average value of the elastic data in the plural sections, and a standard deviation of the elastic data in the plural sections to be displayed in a numeric value on the histogram image, and further displays positions of the plural measurement points in the sections defined by the cursors on the elastic image so as to be identifiable.

**19.** The ultrasonic diagnostic system according to Claim 11, wherein the histogram evaluation means obtains a ratio of the elastic data in the sections of two elastic data designated by two cursors displayed on the histogram image so as to be displayed in a numeric value on the histogram image.

**20.** The ultrasonic diagnostic system according to Claim 11, wherein the histogram evaluation means obtains a half-width of the elastic data of a peak in the histogram distribution designated by a cursor displayed on the histogram image, evaluates a tissue property corresponding to the peak based on the half-width, and displays the evaluation result on the histogram image.

**21.** The ultrasonic diagnostic system according to Claim 9, wherein:

the image generation means automatically changes the interest region set on the elastic image by following the tissue which displaces in accordance with the pressure; and
the histogram evaluation means generates the histogram with respect to the changed interest region.

**22.** The ultrasonic diagnostic system according to Claim 8 or 9, wherein the image generation means reallocates a gradient of the elastic image with at least one of a hue and a brightness corresponding to a width of the elastic data with the histogram distribution in the interest region to intensify a contrast of the elastic image.

**23.** The ultrasonic diagnostic system according to Claim 8 or 9, wherein the image display means displays the histogram on a small window the histogram to be superimposed or as being half-transparent on the elastic image.

**24.** The ultrasonic diagnostic system according to Claim 14, wherein the image display means displays the tomographic image, the elastic image, and the histogram on windows, and is allowed to switch each size of the windows in response to an input command.

**25.** The ultrasonic diagnostic system according to Claim 8 or 9, wherein the histogram evaluation means differentiates the histograms corresponding to the plural interest regions set on the elastic image by different colors, which are synthesized.

**26.** The ultrasonic diagnostic system according to Claim 8, wherein:

the elastic data calculation means obtains a displacement vector of the tissue at the plural measurement points in the tomographic site of the body of the subject based on the ultrasonic tomographic data;
the elastic image generation means generates the elastic image for displaying the displacement vector corresponding to the measurement points in the tomographic site; and

the histogram evaluation means generates the histogram showing a distribution of the displacement vector component in a specific direction in the interest region set on the elastic image.

27. The ultrasonic diagnostic system according to Claim 26, wherein the interest region is set to a region which contains a boundary between different tissues.

28. The ultrasonic diagnostic system according to Claim 8, wherein:

the elastic data calculation means obtains plural elastic frame data on plural tomographic surfaces of the body of the subject based on ultrasonic tomographic data measured by changing the measurement tomographic surface of the body of the subject; and

the histogram evaluation means generates a histogram showing the elastic distribution of the plural elastic frame data.

FIG. 1

# FIG. 2

START

↓

OBTAIN ULTRASONIC TOMOGRAPHIC
DATA UNDER PRESSURE — S1

↓

SELECT ONE PAIR OF FRAME DATA — S2

↓

CALCULATE DISPLACEMENT VECTOR AT MEASUREMENT
POINTS BASED ON THE PAIR OF FRAME DATA — S3

↓

CALCULATE ELASTIC MODULUS BASED ON
DISPLACEMENT/STRESS AT MEASUREMENT POINTS — S4

↓

DISPLAY ELASTIC IMAGE — S5

↓

SET INTEREST REGION — S6

↓

GENERATE AND EVALUATE HISTOGRAM — S7

↓

DISPLAY HISTOGRAM IMAGE DATA — S8

↓

END

EP 1 938 754 A1

FIG. 3(A)

FIG. 3(B)

EP 1 938 754 A1

FIG. 3(C)

FIG. 3(D)

EP 1 938 754 A1

## FIG. 4

**20** HISTOGRAM EVALUATION UNIT

| **13** | **31** | **32** | **33** | **34** | **9** |
|---|---|---|---|---|---|
| DISTORTION/ ELASTIC MODULUS CALCULATION UNIT | MEMORY UNIT | HISTOGRAM CALCULATION UNIT | STATISTIC EVALUATION UNIT | IMAGE DEVELOPMENT UNIT | SELECTOR ADDER UNIT |
| ELASTIC FRAME DATA | ELASTIC FRAME DATA | HISTOGRAM DATA | HISTOGRAM DATA | HISTOGRAM IMAGE DATA | |
| | | | STATISTIC EVALUATION DATA | | |

SYSTEM CONTROL INTERFACE UNIT

ROI COORDINATE DATA

CONTROL SIGNAL

**17**

EP 1 938 754 A1

## FIG. 5

ELASTIC FRAME DATA $\quad X_{i,j} \; (i=1,2,3,\cdots N,\; j=1,2,3,\cdots M)$

HORIZONTAL AXIS DIRECTION OF IMAGE

VERTICAL AXIS DIRECTION OF IMAGE

| $E_{1,1}$ | $E_{2,1}$ | $E_{3,1}$ | $E_{4,1}$ | $E_{5,1}$ | $E_{6,1}$ | $\cdots$ | $E_{N,1}$ |
|---|---|---|---|---|---|---|---|
| $E_{1,2}$ | $E_{2,2}$ | $E_{3,2}$ | $E_{4,2}$ | $E_{5,2}$ | $E_{6,2}$ | $\cdots$ | $E_{N,2}$ |
| $E_{1,3}$ | $E_{2,3}$ | $E_{3,3}$ | $E_{4,3}$ | $E_{5,3}$ | $E_{6,3}$ | $\cdots$ | $E_{N,3}$ |
| $E_{1,4}$ | $E_{2,4}$ | $E_{3,4}$ | $E_{4,4}$ | $E_{5,4}$ | $E_{6,4}$ | $\cdots$ | $E_{N,4}$ |
| $E_{1,5}$ | $E_{2,5}$ | $E_{3,5}$ | $E_{4,5}$ | $E_{5,5}$ | $E_{6,5}$ | $\cdots$ | $E_{N,5}$ |
| $E_{1,6}$ | $E_{2,6}$ | $E_{3,6}$ | $E_{4,6}$ | $E_{5,6}$ | $E_{6,6}$ | $\cdots$ | $E_{N,6}$ |
| $E_{1,7}$ | $E_{2,7}$ | $E_{3,7}$ | $E_{4,7}$ | $E_{5,7}$ | $E_{6,7}$ | $\cdots$ | $E_{N,7}$ |
| $\vdots$ | $\vdots$ | $\vdots$ | $\vdots$ | $\vdots$ | $\vdots$ | | $\vdots$ |
| $E_{1,M}$ | $E_{2,M}$ | $E_{3,M}$ | $E_{4,M}$ | $E_{5,M}$ | $E_{6,M}$ | $\cdots$ | $E_{N,M}$ |

EP 1 938 754 A1

# FIG. 6

EP 1 938 754 A1

## FIG. 7

ELASTIC FRAME DATA   $X_{i,j}$ $(i=1,2,3,\cdots N, \ j=1,2,3,\cdots M)$

HORIZONTAL AXIS
DIRECTION OF IMAGE

| $E_{1,1}$ | $E_{2,1}$ | $E_{3,1}$ | $E_{4,1}$ | $E_{5,1}$ | $E_{6,1}$ | $\cdots$ | $E_{N,1}$ |
| $E_{1,2}$ | $E_{2,2}$ | $E_{3,2}$ | $E_{4,2}$ | $E_{5,2}$ | $E_{6,2}$ | $\cdots$ | $E_{N,2}$ |
| $E_{1,3}$ | $E_{2,3}$ | $E_{3,3}$ | $E_{4,3}$ | $E_{5,3}$ | $E_{6,3}$ | $\cdots$ | $E_{N,3}$ |
| $E_{1,4}$ | $E_{2,4}$ | $E_{3,4}$ | $E_{4,4}$ | $E_{5,4}$ | $E_{6,4}$ | $\cdots$ | $E_{N,4}$ |
| $E_{1,5}$ | $E_{2,5}$ | $E_{3,5}$ | $E_{4,5}$ | $E_{5,5}$ | $E_{6,5}$ | $\cdots$ | $E_{N,5}$ |
| $E_{1,6}$ | $E_{2,6}$ | $E_{3,6}$ | $E_{4,6}$ | $E_{5,6}$ | $E_{6,6}$ | $\cdots$ | $E_{N,6}$ |
| $E_{1,7}$ | $E_{2,7}$ | $E_{3,7}$ | $E_{4,7}$ | $E_{5,7}$ | $E_{6,7}$ | $\cdots$ | $E_{N,7}$ |
| $\vdots$ | $\vdots$ | $\vdots$ | $\vdots$ | $\vdots$ | $\vdots$ | $\ddots$ | $\vdots$ |
| $E_{1,M}$ | $E_{2,M}$ | $E_{3,M}$ | $E_{4,M}$ | $E_{5,M}$ | $E_{6,M}$ | $\cdots$ | $E_{N,M}$ |

VERTICAL AXIS
DIRECTION OF IMAGE

NUMERIC DATA OF ELASTIC
MODULUS WITHIN ROI

EP 1 938 754 A1

## FIG. 8

TISSUE 1    TISSUE 2    TISSUE 3

AVERAGE : ○ kPa
STANDARD
DEVIATION : ○ kPa
MEDIAN : ○ kPa

ELASTIC
MODULUS
(kPa)

0    100    200    300    400    500    600    700

EP 1 938 754 A1

# FIG. 9

DISPLAY IMAGE

TOMOGRAPHIC IMAGE

ELASTIC IMAGE

HISTOGRAM IMAGE

ELASTIC MODULUS (kPa)

700
600
500
400
300
200
100
0

AVERAGE : ◯ kPa
STANDARD DEVIATION : ◯ kPa
MEDIAN : ◯ kPa

ELASTIC MODULUS (kPa)

0   100   200   300   400   500   600   700

FIG. 11

FIG. 10

31

# FIG. 12

CURSOR

C1    C2

N : ○ counts
m : ○ kPa
σ : ○ kPa

ELASTIC
MODULUS
(kPa)

0    100    200    300    400    500    600    700

# FIG. 13

DISPLAY IMAGE

TOMOGRAPHIC IMAGE

ELASTIC IMAGE

HISTOGRAM IMAGE

ELASTIC MODULUS (kPa)

700
600
500
400
300
200
100
0

FEEDBACK TO ELASTIC IMAGE DATA

41

C1    C2

N: ○ counts
m: ○ kPa
σ: ○ kPa

ELASTIC MODULUS (kPa)

0   100   200   300   400   500   600   700

# FIG. 14

EP 1 938 754 A1

# FIG. 15

ELASTIC IMAGE

COMPRESSION

COMPRESSION

t0

t1

t2

TIME t

P1(t0) P2(t0) P3(t0) P4(t0)

P1(t1) P2(t1) P3(t1) P4(t1)

P1(t2) P2(t2) P3(t2) P4(t2)

HISTOGRAM IMAGE

EP 1 938 754 A1

# FIG. 16

ELASTIC IMAGE · COMPRESSION · COMPRESSION · TIME t

t0 · t1 · t2

P1(t0) · P2(t0) · P3(t0) · P4(t0)

HISTOGRAM IMAGE

# FIG. 17(A)

COMPRESSION

ROI  TUMOR

DEPTH DIRECTION

DEPTH DIRECTION
COMPONENT OF
DISPLACEMENT

# FIG. 17(B)

ROI

COMPRESSION

DEPTH DIRECTION
COMPONENT OF
DISPLACEMENT

# FIG. 17(C)

ROI

COMPRESSION

DEPTH DIRECTION
COMPONENT OF
DISPLACEMENT

EP 1 938 754 A1

# FIG. 18 (A)  FIG. 18 (B)  FIG. 18 (C)

## FIG. 19

ELASTIC
MODULUS (kPa)

ELASTIC
IMAGE

HISTOGRAM
IMAGE
DISPLAYED ON
SMALL WINDOW

700
600
500
400
300
200
100
0

AVERAGE : O kPa
STANDARD
DEVIATION : O kPa
MEDIAN : O kPa

ELASTIC
MODULUS
(kPa)

0  100  200  300  400  500  600  700

EP 1 938 754 A1

# FIG. 20

ELASTIC IMAGE

HISTOGRAM IMAGE DISPLAYED ON SMALL WINDOW

AVERAGE : O kPa
STANDARD DEVIATION : O kPa
MEDIAN : O kPa

ELASTIC MODULUS (kPa)

0   100   200   300   400   500   600   700

ELASTIC MODULUS (kPa)

700

600

500

400

300

200

100

0

## FIG. 21

ELASTIC IMAGE DISPLAYED
ON SMALL WINDOW

AVERAGE : ○ kPa
STANDARD
DEVIATION : ○ kPa
MEDIAN : ○ kPa

HISTOGRAM
IMAGE

ELASTIC MODULUS
(kPa)

0    100    200    300    400    500    600    700

EP 1 938 754 A1

FIG. 22

ROI 1
(PERIPHERAL EDGE)

ROI 2(CENTER)

ELASTIC IMAGE

HISTOGRAM
IMAGE

EP 1 938 754 A1

# FIG. 23(A)

ELASTIC MODULUS (kPa)

700
600
500
400
300
200
100
0

FAT

TUMOR

MAMMARY GLAND

ROI

GREATER PECTORAL MUSCLE

DISPLACEMENT IN DEPTH DIRECTION

AVERAGE :0.05(mm)

LATERAL DISPLACEMENT (mm)

-0.03   -0.02   -0.01   0   0.01   0.02   0.03

# FIG. 23(B)

ELASTIC MODULUS (kPa)

700
600
500
400
300
200
100
0

FAT

TUMOR

MAMMARY GLAND

ROI

GREATER PECTORAL MUSCLE

DISPLACEMENT IN DEPTH DIRECTION

AVERAGE :0.05(mm)

LATERAL DISPLACEMENT (mm)

-0.03   -0.02   -0.01   0   0.01   0.02   0.03

EP 1 938 754 A1

FIG. 24(A)

FIG. 24(B)

EP 1 938 754 A1

# FIG. 25 (A)　　FIG. 25 (B)　　FIG. 25 (C)

PROSTATE CANCER

PROSTATE GLAND TISSUE

ROI

C1　　C2

ROI

ROI

ELASTIC MODULUS (kPa)

ELASTIC MODULUS (kPa)

ELASTIC MODULUS (kPa)

EP 1 938 754 A1

FIG. 26(A)

FIG. 26(B)

FIG. 26(C)

<table>
<tr><td colspan="2">**INTERNATIONAL SEARCH REPORT**</td><td>International application No.</td></tr>
<tr><td colspan="2"></td><td>PCT/JP2006/320221</td></tr>
</table>

A. CLASSIFICATION OF SUBJECT MATTER
*A61B8/08*(2006.01)i

According to International Patent Classification (IPC) or to both national classification and IPC

B. FIELDS SEARCHED

Minimum documentation searched (classification system followed by classification symbols)
A61B8/00-8/15

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
Ichushi WEB

C. DOCUMENTS CONSIDERED TO BE RELEVANT

| Category* | Citation of document, with indication, where appropriate, of the relevant passages | Relevant to claim No. |
|---|---|---|
| X<br><br>---<br>A | Hiroshi KANEI et al., "Kekkanheki no Danseiritsu Dansozo Imaging", Eizo Joho Medical, 2005 Nen 8 Gatsu 1 Nichi, Vol.37, No.9, pages 904 to 909 | 1-6,8-11, 13-15,23-25<br>---<br>7,12,16-22, 26-28 |
| A | JP 2004-351062 A (Hitachi Medical Corp.), 16 December, 2004 (16.12.04), Full text; all drawings & EP 1629777 A1 | 7,26 |
| A | WO 2005/020821 A1 (Matsushita Electric Industrial Co., Ltd.), 10 March, 2005 (10.03.05), Full text; all drawings & EP 1661519 A1 | 1-28 |

☒ Further documents are listed in the continuation of Box C. ☐ See patent family annex.

| | | |
|---|---|---|
| * | Special categories of cited documents: | |
| "A" | document defining the general state of the art which is not considered   to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| | |
|---|---|
| Date of the actual completion of the international search<br>09 January, 2007 (09.01.07) | Date of mailing of the international search report<br>16 January, 2007 (16.01.07) |
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

| International application No. |
|---|
| PCT/JP2006/320221 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2005-270341 A  (Hitachi Medical Corp.), 06 October, 2005 (06.10.05), Full text; all drawings (Family: none) | 1-28 |

Form PCT/ISA/210 (continuation of second sheet) (April 2005)

| INTERNATIONAL SEARCH REPORT | International application No. |
|---|---|
| | PCT/JP2006/320221 |

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:
    See the extra sheet of Box No. III.

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

<table>
<tr><td>**INTERNATIONAL SEARCH REPORT**</td><td>International application No.<br>PCT/JP2006/320221</td></tr>
</table>

Continuation of Box No.III of continuation of first sheet(2)

The technical matter common to the inventions of claims 1-28 is the one stated in claim 1. However, the search has revealed that the above matter is not novel since it is publicly known (for example, document, Hiroshi KANEI et al., "Kekkanheki no Danseiritsu Dansozo Imaging", Eizo Joho Medical, 2005 Nen 8 Gatsu 1 Nichi, Vol.37, No.9, pages 904 to 909).

Therefore, the constitution cannot be considered as a special technical feature within the meaning of PCT Rule 13.2, second sentence.

Consequently, the inventions of claims 1-28 obviously do not satisfy the requirement of unity of invention.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5317313 A
- JP 2000060853 A
- JP 2005013283 A
- JP 2005066041 A
- JP 2004261198 A
- JP 2004135929 A

**Non-patent literature cited in the description**

- **UENO E et al.** Dynamic tests in real-time breast echography. *Ultrasound Med Biol.,* 1988, vol. 14, 53-57